# EUROPEAN PATENT APPLICATION

(11) **EP 2 357 225 A1**
(43) Date of publication of application: **17.08.2011**
(21) Application number: 10305145.4
(22) Date of filing: 11.02.2010
(51) Int. Cl.: C12N 9/12, C12N 15/54, C12N 15/63, C12N 1/21, C12Q 1/68, C12P 19/34, C12P 21/02

(54) **Thermostable primase/DNA polymerase of the Thermococcus nautilus 30/1 plasmid pTN2 and its applications**

(71) Applicant: INSTITUT PASTEUR, 75015 Paris (FR); UNIVERSITÉ PIERRE ET MARIE CURIE (PARIS VI), 75005 Paris (FR); UNIVERSITE PARIS-SUD 11, 91400 Orsay (FR)
(72) Inventor: Forterre, Patrick, 91190 Gif S/Yvette (FR); Sezonov, Guennadi, 75005 Paris (FR); Desnoues, Nicole, 93330 Neuilly-sur-Marne (FR); Soler, Nicolas, 91400 Orsay (FR); Van Tilbeurgh, Herman, 91190 Gif S/Yvette (FR); Keller, Jenny, 75018 Paris (FR); Marguet, Evelyne, 91190 Gif S/Yvette (FR)
(74) Representative: Ahner, Francis

(57) **Abstract**

The present invention is directed to a thermostable primase/DNA polymerase protein of the thermococcus nautilus 30/1 plasmid pTN2 and the nucleic acid encoding said primase/DNA polymerase. The invention also relates to method of synthesizing, amplifying or sequencing nucleic acid implementing said primase/DNA polymerase protein and kit comprising said DNA polymerase protein.

SEQUENCE LISTING

<110>  INSTITUT PASTEUR
       UNIVERSITE PIERRE ET MARIE CURIE (PARIS VI)
       UNIVERSITE PARIS-SUD 11

<120>  THERMOSTABLE PRIMASE/DNA POLYMERASE OF THE THERMOCOCCUS NAUTILUS
       30/1 PLASMID pTN2 AND ITS APPLICATIONS

<130>  D27760

<160>  29

<170>  PatentIn version 3.3

<210>  1
<211>  13015
<212>  DNA
<213>  artificial

<220>
<223>  Thermococcus nautilus 30/1 plasmid pTN2 nucleic acid sequence

<400>  1
   gtgaccgctc cctactttcc ctacactatc aaactttttg gccctccagg gacgggaaaa      60
   actagaacgc ttcagaaact taccgaatgg ctcattgggg agggagaaga gaataaagag     120
   gaactcatag ccaaaggatt tcccaaggaa tggctggaag agaactttgg aaaatacgcc     180
   ctctctgaga ttgctttcgc taccttccag acctctgccc taaaagagtt tgtcgaggat     240
   cgtcttggca agagcctcaa gaacgataga gcccccggag gggatcttag gtacttccga     300
   acaatccacg gaatttgcca ggttctccta agagatgccg ggctggatgc atggaagtca     360
   gtctcacgaa aaatgggagg tctcagtccc gagcggtggt ttcagatatt tgccaatcgc     420
   agaaggaaag aagtggatcc gagtttccgt tttgaccccg attctatggg cgccgccaac     480
   cccttcgaaa aagcgaacta cctctggcag gtcaaaagcc aagttatcaa tcgtgaatac     540
   cctaaggttg gccgagagca cattactgag agagttctgg aagagctccc tccaagttta     600
   caggatcatt accttgcttg gctcgatttc aaacaggaaa agggcatcaa agactatgac     660
   gatatgctaa tagatgccta cgatgccctc aaagccggtg tgataagcct gcccaccaga     720
   gttctcatcg tggacgagtt ccaggacctc agtccgttgc aattcgagat tttcaaaatg     780
   ctcgcaagag ataaggagct cgtggtcatc gccggcgatg attggcagac cattttcact     840
   tggatgggtg cggatccaag attccttgtt aattaccctg ctgacttgca gataattcta     900
   agaaagacgt ggaggctccc tgagaaaatt cttcgcgaag cccttaccta cgcaaaggct     960
   aaccttcgga gtggggtatt taaagagatg gtcagcacag gaaagagggg ggctattgga    1020
   atagtttcca atttctccac taaaaatgat aatgacttgg acctcttggc gaggtgggtt    1080
   ctcaacgagt tgaagcgggg ccactctgtt tttgtcctcg ccaggacaaa ccggcaggct    1140
   ctacacatct tcggcgaact ctacaagaga ggattcaagc ccaagaatct caagaagtcc 1200
   agccgctgga agaaaagagt agagcctgtt ggggatttct ttgatgtgct tcatgccatc 1260
   gtggcaattg aaaggggaac cccctcacga gaagacttta agagagttgg gtgggcctgt 1320
   ggggttatta gcgaaaaaga gtttgagaaa gaccttccct tgatggagta cattaagccc 1380
   gactggaaga ggactattaa acttgagcgg attcaagaga tttgggggcg cacagcaagg 1440
   cgtttcctag aaaagattct caaggaaggt atcaccccca tagcgggtat tcctgaggac 1500
   cacgagcttt atgtggatac tattcacagc tccaagggtc tcgaagctga cacagtcttc 1560
   cttatcaacg aaatgccgag aagaaactgg aggagatact tcaaacggcc agaggatctt 1620
   gaggctgagg cccgtgtgtg gtttgttggg atgactcgcg cacggaaggg ccttctcata 1680
   atcaacacga ataaagcctt ccacctgtag cggaggtgtt tgagataccc ggctacacgg 1740
   tccaagtaga gaattggagg taatgctcgt acggggggat ttgtatggtg gagattattg 1800
   cccctccgga tgcgtttcga acgtcgtcag ttgagattga gcctccacgg acgaaggtga 1860
   gccggttgcc cactcctcct cggggaattg ccgcagaacc gaggcagatt ccaaccggtg 1920
   gtgtcgtgaa gttcgaggaa atagggttgg atgaggagta tctcaaaaag cttgagaagt 1980
   ttgcaaagga ggttgggctt gaggtccctg agaagctcaa gagaaaatat cgggtgcgtt 2040
   ctgagggggc tgaggccgtt aaagagatgc tccggagcga gctcaacaac tttcttgaca 2100
   agctcaagaa gaaatacgag gagaagctct ccaacgagaa gaagagctct caggttacac 2160
   cttcatcaca gtcctctgta gaacagtcct cctcgggtga tgctgtggat tcagaggctc 2220
   agcaaatcct tagttacgag attggcctgt cgcaggagga catcacaaag cttgagagtg 2280
   cggcgaagaa ggtggggttg caggtccctg agaatctgaa acagcatcgg gtggtttcgg 2340
   ctgaggagaa agatgaggct ttagcagagt ttcagaagga gctcgatgag tttctcgaag 2400
   agttacgtac ggcttacgcg aagcagtctt atgagcggga tatgatgcgg ttgaggggga 2460
   tggctgaggc gtacaaggaa aagcaccccg gaagtcgcgt tttattaacc gacgccggct 2520
   ttatggtcgt aattgacccc gtatcagcgg cgccaccctc ctcggataat tcagagttaa 2580
   atcttgacgt ggacgttagt gaaaagccca tctcaacgcc cgaaccttcc gagagcgaca 2640
   atccatatcg tgggtatgag gagtactaca agggccttta tgctttgagg aaagcgcttg 2700
   acctcgaaac gaacagcaca cttaatcttg agcctgtccc tcgcccgccg aattaccttc 2760
   tcactgacca aaccgcgacg tgggaggata tccagcggac gaacaaagcg atgcacgaga 2820
   tatggacact tggaggcttt ctctcggata aattagggtt atcccctgat aagagcccat 2880
   ctggacggac tgtaaaggtt tacgagagtg agttaagtgc gttggacgta ttgggctccc 2940
   ctgcaacttc gattggggca gacctcgaca gtgaggagga taaagttttt gcagaggccg 3000
   acttggaaaa gcccgttgag gagtttctca agggtgttgg taggggtgct attggcgttt 3060
   tcacattccc ggccgatatc gctttgtttg caatgcaagc ggacaagctc aagtatttag 3120
   ccgccgaaga gaacaagagc gttggggaat tcttgaaaga ccaggcaata gacgccctcc 3180
   atgacccggg cttttatgga gagctcttcg gttcgttgtt aacctcggcc gggctttcct 3240
   ctctcaagaa cgcaatgaag ccgaaagtgt acgtggagaa aggcggggtg cagggtgtta 3300
   ggaccacaat cgaaaaactc aacctcgatg gggatgaact tgcccgtgct gtgagaaccg 3360
   agctctttga cttagataag accacaacaa tcaccgttca gaaaaagtac aagttattcg 3420
   ggcctaaagt catcaatgtg gatacaactt atgacagctt caaggctatt agctccgagg 3480
   ggataaaaac aagttcagga aacgtgatgg gcagaacgag gatactggag ttctcgaacc 3540
   ttgatgatgg gctgaatgta ggcaaattca cagtacgtga gctcgccaag atgggagatg 3600
   actccgccag gctcttctca gaggcacaga aagacatagt agcctcaacg gatgacatcc 3660
   tttccttcgt ggatgaggca acctccagtg gaaagagtgg gccaacgaaa attgtgagaa 3720
   ccaccgacta catcacagat gagggcttga aaataaagac tctccaagag ttctatgatg 3780
   acatagtgaa aaccgagcag aagattacct cattcgcaaa accaaggccc gtaacagggt 3840
   cgaagaaggg gagctccttc tcaacgttag acgacctagt gaacaaagcc gcttgtgatt 3900
   tggacgaggt aataaagagc gtagatacca gaaatcccat aggaagtggt ggagtaacgg 3960
   ttttagaaga agaccccctc aaggggctta ctgacttggg gttcaaggtt agagaaaagc 4020
   cgactacacg gactgaggtg acaccaaaat cttcccccgt agccggcttc aaagttccgg 4080
   atctctccac cgaggtggaa aatatcctta atcctaatct ctcagtggga gactttgaac 4140
   aggaccagaa cacaggttct acaaagcgct cccgtcgtag ggaggttcaa aagacaatct 4200
   cccacccaga tatcagggta ctcagcttgg gagaacttga agaaagtgta tttggcataa 4260
   acacctcacc gagggttaga atttcaagta agacaagttc cgggcagaga gattaccgca 4320
   tccccactca agtagtcaca ccaaaagaat ttcacataga tgagccgaag tgggatatcc 4380
   gggacataac agacccactt gaggacattg acagagaaac gcgggcgatt ctctcacagg 4440
   ttctcgatac ccggctcacc accaaaacaa acccgaaaaa accgaaatac aagacaaaga 4500
   aaaagaagaa gaggccgata caaacagcac caaagaagaa gaaaagccaa aagaagagaa 4560
   ccaaaggcaa gggtggtgaa tggggaatct ggattggata cgaaagcctt ttggactggt 4620
   gatgcttcga gggcgttctt tcactctcca accacttttt gagtttacag actccgcata 4680
   aattcaatta gataaaaata cacccggtgg gagaggagac aacggggagc gctccttcct 4740
   gcattcaccc caaagaaggc gccagaacca atgtttttct cacccacttt ggccggtact 4800
   tttaatttaa cttccgatgt aagccttatg ggggatgacg atgtctctaa aatcccgact 4860
   tcgcgctttc cttggcctcc ctgaagtgga ggctgagatt cgagaaataa aggagactct 4920
   ctccaaactc tccgatgcac ttgaggacct ccaatccaaa gtagatgctc aagatgaaca 4980
   acttaagaga gccctcaccg agttgagccg caaggctgat atgggattgt actacgagct 5040
   aaatgaacgt ctcgaaacta cgagagatga aataaaggct atggaagcgg aaataagctc 5100
   aatctgggat aaggtgaagt ttatcgaatt ctcgttctcc gagcattccg aagagcctga 5160
   aatgaccgtt gaagagcttg caagcatcgt cgaacattac attcgtaaag gtctaacccg 5220
   cccatcagaa ctcaagaaag ctgttggggt ttcgtgggaa aagctctatg cggcccttac 5280
   ttacctcaaa ctaaagaaga gaatagcgag aattcaaaag ggccgtagag tcgaatatgt 5340
   tttagtggag gaagccgaag aataattctc ggccatgctc ggcccccgag aagtcttatt 5400
   tctcttcccc caattttcaa ctttttgacg tgattttctc caagaagtct ctcccccaca 5460
   accacggttc tacacgaggg agtatttaaa taagacatac ttatgtcatt aatagggttc 5520
   ccaaccccat tgggaacggg gggctctaaa gccttcaatc gaaaacgtta aaagggtggt 5580
   gtcgtaatta tgacacgcaa gacatattta gggcacggtg ggactatgat tgacagcatc 5640
   atcgaaaagc tcgtagaaat gggggccttc ccggcccgca agaagacaaa aaccgagacc 5700
   atcagagtcg tctcaaacat gctcgaagcc atcaaatacg aatacaacgg ccggctacca 5760
   cggtgggagc tcaaaacctt tgtcacacgc tttgctaacc aatcaaagtt ctccgagcga 5820
   caaatctaca ggtacataaa cgagctgaaa gcattgggct ttctcgaaga catagtagac 5880
   gtaggaaccc agaaacatta catcgttctc tcaaagcgct ttggttcccg gatgcttgcc 5940
   ctttaccggg actacaacaa atggttaggg gaggtgtaac cgatggtgcg gcccttcaaa 6000
   gttgtggtgg ttggaaaaac cgagaatgag aagatatggg agttagaggc cctcgtgttt 6060
   gcaaaggacc aaactcaagc agaagagctt agcctcacca ctgccaaaaa aagagggctc 6120
   caaaccccca aagtggtgta catgaaagaa taccgcaggg caagagtcta cgcaatcggc 6180
   aaattcggat ggcccgacga ctgggtatgg gacctcatag aagaaggtct tggccaagct 6240
   ccagccgggc tcgtttcact agaggacata gaggtgtaaa acaaatgagg gactcaagat 6300
   attcttcgtg ggtcgagaaa aaactacacg aggacctcac aagatattac acccacagaa 6360
   cgctggacgt ctatgacatc tggctgatta tgaacctcct ctatgatgga acaacaatgc 6420
   atagcctcga aatcgagaga gcaggaggga aaacctactt cacgctagac accccatcca 6480
   tctcatatgg agtttatgac ccagagaaga ggaccttcac cacaaagtac gagcacattc 6540
   caggaccaca cctgaaccta aaagaaatcc acctcaacat cccggaatcc atagagaaac 6600
   taaaagtgaa aggaaaagag aaatggctaa tcacacgccg aaaccccgtg aattgggatt 6660
   atgccccccg attcaagcct aacatttaca tacaaggccg aatctggagc gatgacggag 6720
   taaaagggtg gcttcaacga aaagaagatg agttcgccta cgtcgcaatc ctcggaacga 6780
   tagttacccc ctacgtatta ggagaagacc caaaaccggt attctatgaa agagaagtct 6840
   tcatcgctca aggggccttt ttgggagaca ccatccaact catccacaat ttcccgcaca 6900
   gccccaagat aatcccccgc agagtcattg aacaactcca actaaaggag gggttatgat 6960
   ggcagatgcc gtgcttttag aaaatatcta cttcgcccta agccaagccg acagggaaac 7020
   aaccgcaagg gggaaaatcg ggtatctcct aaccgccgcg tcccttttag aggccctcgt 7080
   cgaaaaagat agactaacag gcccaataac aattacaaaa gtgaaatatg agctcgaaca 7140
   agacgagagg ggcaactaca aaagccggag accaagcgaa gtggagaggg taacactgaa 7200
   tacaacagat gagataaccc ttttcattga aaaacacatc ataagctctc taatacaagt 7260
   tgcaagcctt ccccacaccg caatgttaga caaagtgtgg gaattgggct acaaacccga 7320
   ttggggggaa ctacgcacaa ttcaaatttt tagctacatc gaggaaatca aaagccttct 7380
   tttgaaattg ctcgtcgcgc tcgacctcgc gaacgtcgca ttagtagaca tagcaactaa 7440
   gctagagaaa tccacccgta gaaataccac tacataagtt gaattaaaca aaatggggga 7500
   gggatagtga attcatacat acacacctgg aggtgccgag cgtgagtagc gtgaaaggca 7560
   tgcaaatcca cgcccgcaaa ggagatgaac tcctcgaaaa gctactctca caagaccccg 7620
   cttatggagg tagacaagcc gccatctacg gagccgttgg aagcggaaag acaaccctcc 7680
   tactctggct tgcccaagtc ttacaagccc gcggagagtg gataatctgg agaggaagag 7740
   acaccgacgg ctggcacatg ctccacagct ggaagcaaag agtaaaactc ctcgtccacg 7800
   agaaagacga agtccacgtc tacaaagtcc cctacggagg caccaccgcg accgaaatca 7860
   cgaagagttt gaacatcgag aagtacagta ccccagagga cctcctcaca aaacttgaac 7920
   aagacaaaat caacatcgtg tacgagccca ccttttacca gataacccaa gagctcgcca 7980
   cttacatcct cgccagaacc aacatccgga taccatcttg gcagatgaaa gaggccaaag 8040
   gcatttactt ttggtttgag ctcttcgaga aactcatcac taggaaagac cgcagatgga 8100
   taaccatcat cgtagacgag gccgatgacc tcatcccagc taacccccag ggcctccagt 8160
   ggaggctaca ggaatgggta aagaactccg tcaaggactt cagaaagaac ctcatcaccc 8220
   taattctcgc ggtccacaac gccgaggact gcgaccaccg cgtaatcagg aagctcccca 8280
   tccggattta cttacaggga gcaagagtgg acaaagacag cctgatgacc gacaaaacaa 8340
   tcacccaacg tctcaacccc ggagaagcaa aaatcgaatg gagaggcttc ggatacggag 8400
   gcttcaaatt ccctccacta gaaaggcgcg attacgccat tatcgtcgag aagaaatgga 8460
   ccggagaact ccccaaattc gaagaagata agcgaaccgc agggagcatc atccaagaac 8520
   tccgcgaaat cgccgaggaa gaaggcatcg aagaagccct cgaacgcgca tgggaactcg 8580
   tccaagaggg agtcatcaca aaacgctact actaccaaat ccgcaaagcc ctccgcgaag 8640
   aaagctgact cttctttcaa cttttgtgaa ggccctcaaa gtgaacagag atgttcaccc 8700
   cctcatctgt gttcggaatc ttgatattcc gcaaaattca aacttcacat aataaaaacc 8760
   atattagaat tgcaggttaa agtactcatt tgttcaatat tactcatcta tgtcataccg 8820
   agagagacgc atggtcggtt accattagaa atacgaacac aaaatggtgg ctgaagtggc 8880
   taaagtggct gaagtggcta aagtggctga agtggtcaaa agggttgaag tgttctcaaa 8940
   aactagtcca atgaaactcc cttctaaggc ctttttttgc tgaaaaaaaa gagttatcac 9000
   attttttctc tacgtctctc ttcaaaattt ttgaccactt tgaccacttt gaccactttg 9060
   accaccgcga ccactacaac cccccttcct aatatgcttg ggataatatc atacacacca 9120
   aacaaaaagt atttaaatag gtgtgtatta aattatgcca gcaaacggaa gggggtactc 9180
   ataatgaatc cctcaattga ggaaatcaaa cgccgtttga ttcaagctgg tgcatcgcca 9240
   caagcagtcg agagccttga accggaattc tttgaggatc ttactgaaga catggacttt 9300
   gaggggactg ctagggttat taatttcata gactatctcg aaaactttga gaattacaag 9360
   agaaagcgag tgaatattac ccttgctgtg cccgtgtatg aggttctaaa gcacatcgca 9420
   agcaagattg ttgacgctga tggtcgtcca taccccgtga gttatcttat cgaggacata 9480
   attgtttggg ttctgaaaga tccagatagg tttgtgcaat ttgtggagga aacttacccg 9540
   gaggaggata atgatgagag cgaggagaca cactcggaga ttgaagagca agcgtaaggt 9600
   gggtgtagga cgccctccag gacgaagaaa ggtgaatcac ctagatgtaa gcgctctcag 9660
   cgcggagtgg aaacagttgc tagatgtggt tgggcttggg agaaagaaaa gaaagggcct 9720
   ttcatactga agttagtccc cccacagatg gaggtgatgt aaatgtcccc caatactaag 9780
   tgctcggttc cccttaaaaa atcaccgtat ccttgtgaga gtgttaatgc aggagccaca 9840
   attcctaggg atatctacac cttgctcgaa gaacttaggg cgcttgacgt tttgaatgcc 9900
   aagaattgga aggagattta cctagctctc ttgagaaatc ttacggagct taatgagcgt 9960
   tggaagctct tggcattaat ttatcgcatc gttgtatttc atagcgaccc ctccacggca 10020
   agtacggtcg ttggcaagtt tctcaagaat tttgacattc tatttgaaaa actccccggc 10080
   caccgggctt atttcgcccc cactcaggtg gggatgtggc tttttgagaa tcttatcaaa 10140
   cagggagggc agggagagat agagttcagg gctgagcgcc aaacgtcagt tgtgcagcag 10200
   ctatccttaa tgtccttcct ccagaacacc cctaaggggg tgaaagtatg agcagtcttc 10260
   gtccatcttc aataatcatt gatatctaca aggttattca ggatcaccca gattccagcc 10320
   ggtgggctat tgaacttcgc ttccttccga agccgatatc ttcggagtgg attttcatta 10380
   ctgacattga ggaaagagct tctgagattg ataaggttct tactaagtac aacataatga 10440
   agaagaaaga cgcctacgtc tctatggcga tacacgattt cgagaaggtt accgcgaagc 10500
   tgaagcgtgt ccaagaagaa gagaacaaaa aagccacgga gggagagagg aggcttagga 10560
   ggatcaccct agacaaaatc cagggggatt cagaaaccac ctcaggtttc actctcgctc 10620
   ttgtggttga tattgacaat accaagatcc acgacactag gattattgag aatgaggagg 10680
   aggcctttga ggcatcaaaa agagagtggg aggcgcttaa gcctaaactt caagaactgg 10740
   ggttcctccc aagatggatt ctttacacag gcggagggct tcagctctgg tttgtcagtg 10800
   acaaactcga acccatctca gtgattgaca gggcctcgga gattatcccg aacattatga 10860
   atggggtgaa tggcgtcaag gggcttcttt ccgaggggtt caaggctgac aacatctttg 10920
   atcctgctag gatagttagg gcgccgctca ctttcaatca taagtacagg accatcataa 10980
   aggacgaaga cggaacggag agggttgttc ccacacaggt aaaaggtcgc gttattgagt 11040
   ttaacgacgt caggatttcg ctcacggagt tccttgacag gctggaggcc tacgctaaag 11100
   agaaagggat tcctctcgaa aagcccacta agcggaagtt ccttgaactg gcttcaaaga 11160
   gatacgaagt cacgtcctcg aactttgaag ccctcgccga gagactcttc acggagctta 11220
   ggccttggtg ggagatagcc aaggaaaagg gctggtctag gcaccatctc acgatgggca 11280
   tagctactta catcttgagg aacactaatc taaccccgga gcagctcatt ggaagtgaaa 11340
   actcaccggg gctttgggag ctcgtcttcg ccaaacttgt tgaagcagga ctggaagatc 11400
   cggatgattg gaagaacagg gcctccacga taagggacgc ctacaagaag atagagagtg 11460
   gaaagaaggt agcaacgaag gcctacctcc ggaagtacat tgagggcctt tcggaggagg 11520
   atgctgtcca gattctcctc tctgtgaaga gggcactcct cccttaccta aaagcggtgg 11580
   atgtgaagag gatctctaag tacagcgcaa ggccatatga gattacagaa gaaatcccaa 11640
   agagctggga agatattgac gagaatcgta aaaaagcaac tggagtttgg tacatagatt 11700
   tccttgctct cgaaacagcg aatgacattt acttcgagga tcttcctaag cctcccgttt 11760
   tctacattcg gtttgttgaa aagaacaagg agaaatttaa gcttaatgaa actctctatc 11820
   attcctttct gacttggctg gggatcaccg aaggagagcc actcgataga actgagctga 11880
   ttgaccttct cgtggagaaa tttggtttta ctgttgagga cctcaaggct atctactaca 11940
   gaaagatcct cacccttcta aaacctgagg ggatgaggac ccccaagtgc atccaggagt 12000
   tcctctttga gctcgccacg gaaggtaatc tcccagaaga taagattcga cacttggcac 12060
   attgggttaa gttctatgct agaccccttc ggcattcgac cacgtcagta ctccttaaag 12120
   gcagaggcaa gccagtagat atgaggctgg caatttgggc caaagtcgtt gagttcttcg 12180
   ccgaagatga tgaggtagct gaggagttaa taactacctt caagaaagca tacatgggag 12240
   ctgagcctcc cttcccctgc atcggagctg agtcatgccc attctatcct gaccacaggg 12300
   cttgtccgtt catagttcca aagcgcaaag aggttctacc agtgagcatt gtggacgttc 12360
   agttacacgg ctcggatggc attgttgttt tagtcggtgg tccgacagag gtaacagcgt 12420
   ttactctcga aggaaaagtc gagtggataa agaccactaa aaagacagtc aagtatccca 12480
   tagccgagtg gttccttgac aggtttgcca aagagtactt gtctctcccg gaggctcctt 12540
   cttggtggaa gcttgaggag gttactgaga tactgaaaag tcggcgaggg gttgttaaaa 12600
   gccaatttga caagtttgag gactaccttg aacagttcat cgagtggctc cagaaggaga 12660
   attcccgccg ggggatactc ccctacgaaa aggccgatga gaaccactta ttcatcaagg 12720
   gcgaatgggt agggatcccg ccaggatttg ctagggaatt ttacagtggc gaacttctca 12780
   taggtggccc aaccttcagg agaatgctcg aacaaaagct cggaaaggat taccggaaga 12840
   tgtcggcaaa aatccacctc aacacgggcc taaaggacaa gaggaactgc tacttcgtga 12900
   gtgtggagtg gttcagaaag catgtaggcg aacccaacat ccaagagata acctccgagg 12960
   gggatgtctc atttgacggt ctcagctatg acgatgaaga ggagggggtg gtcgg 13015

<210> 2
<211> 1710
<212> DNA
<213> artificial

<220>
<223> Thermococcus nautilus 30/1 plasmid pTN2: ORF1 nucleic acid
 sequence

<400> 2
gtgaccgctc cctactttcc ctacactatc aaactttttg gccctccagg gacgggaaaa 60

actagaacgc ttcagaaact taccgaatgg ctcattgggg agggagaaga gaataaagag 120

gaactcatag ccaaaggatt tcccaaggaa tggctggaag agaactttgg aaaatacgcc 180

ctctctgaga ttgctttcgc taccttccag acctctgccc taaaagagtt tgtcgaggat 240

cgtcttggca agagcctcaa gaacgataga gcccccggag gggatcttag gtacttccga 300

acaatccacg gaatttgcca ggttctccta agagatgccg ggctggatgc atggaagtca 360

gtctcacgaa aaatgggagg tctcagtccc gagcggtggt ttcagatatt tgccaatcgc 420

agaaggaaag aagtggatcc gagtttccgt tttgaccccg attctatggg cgccgccaac 480

cccttcgaaa aagcgaacta cctctggcag gtcaaaagcc aagttatcaa tcgtgaatac 540

cctaaggttg gccgagagca cattactgag agagttctgg aagagctccc tccaagttta 600

caggatcatt accttgcttg gctcgatttc aaacaggaaa agggcatcaa agactatgac 660

gatatgctaa tagatgccta cgatgccctc aaagccggtg tgataagcct gcccaccaga 720

gttctcatcg tggacgagtt ccaggacctc agtccgttgc aattcgagat tttcaaaatg 780

ctcgcaagag ataaggagct cgtggtcatc gccggcgatg attggcagac cattttcact 840

tggatgggtg cggatccaag attccttgtt aattaccctg ctgacttgca gataattcta 900

agaaagacgt ggaggctccc tgagaaaatt cttcgcgaag cccttaccta cgcaaaggct 960

aaccttcgga gtggggtatt taaagagatg gtcagcacag gaaagagggg ggctattgga 1020

atagtttcca atttctccac taaaaatgat aatgacttgg acctcttggc gaggtgggtt 1080

ctcaacgagt tgaagcgggg ccactctgtt tttgtcctcg ccaggacaaa ccggcaggct 1140

ctacacatct tcggcgaact ctacaagaga ggattcaagc ccaagaatct caagaagtcc 1200

agccgctgga agaaaagagt agagcctgtt ggggatttct ttgatgtgct tcatgccatc 1260

gtggcaattg aaaggggaac cccctcacga gaagacttta agagagttgg gtgggcctgt 1320

ggggttatta gcgaaaaaga gtttgagaaa gaccttccct tgatggagta cattaagccc 1380

gactggaaga ggactattaa acttgagcgg attcaagaga tttgggggcg cacagcaagg 1440

cgtttcctag aaaagattct caaggaaggt atcaccccca tagcgggtat tcctgaggac 1500

cacgagcttt atgtggatac tattcacagc tccaagggtc tcgaagctga cacagtcttc 1560

cttatcaacg aaatgccgag aagaaactgg aggagatact tcaaacggcc agaggatctt 1620

gaggctgagg cccgtgtgtg gtttgttggg atgactcgcg cacggaaggg ccttctcata 1680

atcaacacga ataaagcctt ccacctgtag 1710

<210> 3
<211> 2839
<212> DNA
<213> artificial

<220>
<223> Thermococcus nautilus 30/1 plasmid pTN2: ORF2 nucleic acid
sequence

<400> 3
atggtggaga ttattgcccc tccggatgcg tttcgaacgt cgtcagttga gattgagcct 60

ccacggacga aggtgagccg gttgcccact cctcctcggg gaattgccgc agaaccgagg 120

cagattccaa ccggtggtgt cgtgaagttc gaggaaatag ggttggatga ggagtatctc 180

aaaaagcttg agaagtttgc aaaggaggtt gggcttgagg tccctgagaa gctcaagaga 240

aaatatcggg tgcgttctga gggggctgag gccgttaaag agatgctccg gagcgagctc 300

aacaactttc ttgacaagct caagaagaaa tacgaggaga agctctccaa cgagaagaag 360

agctctcagg ttacaccttc atcacagtcc tctgtagaac agtcctcctc gggtgatgct 420

gtggattcag aggctcagca aatccttagt tacgagattg gcctgtcgca ggaggacatc 480

acaaagcttg agagtgcggc gaagaaggtg gggttgcagg tccctgagaa tctgaaacag 540

catcgggtgg tttcggctga ggagaaagat gaggctttag cagagtttca gaaggagctc 600

gatgagtttc tcgaagagtt acgtacggct tacgcgaagc agtcttatga gcgggatatg 660

atgcggttga gggggatggc tgaggcgtac aaggaaaagc accccggaag tcgcgttttt 720

attaacgacg ccggctttat ggtcgtaatt gaccccgtat cagcggcgcc accctcctcg 780

gataattcag agttaaatct tgacgtggac gttagtgaaa agcccatctc aacgcccgaa 840

ccttccgaga gcgacaatcc atatcgtggg tatgaggagt actacaaggg cctttatgct 900

ttgaggaaag cgcttgacct cgaaacgaac agcacactta atcttgagcc tgtccctcgc 960

ccgccgaatt accttctcac tgaccaaacc gcgacgtggg aggatatcca gcggacgaac 1020

aaagcgatgc acgagatatg gacacttgga ggctttctct cggataaatt agggttatcc 1080

cctgataaga gcccatctgg acggactgta aaggtttacg agagtgagtt aagtgcgttg 1140

gacgtattgg gctcccctgc aacttcgatt ggggcagacc tcgacagtga ggaggataaa 1200

gtttttgcag aggccgactt ggaaaagccc gttgaggagt ttctcaaggg tgttggtagg 1260

ggtgctattg gcgttttcac attcccggcc gatatcgctt tgtttgcaat gcaagcggac 1320

aagctcaagt atttagccgc cgaagagaac aagagcgttg ggaattcttt gaaagaccag 1380

gcaatagacg ccctccatga cccgggcttt tatggagagc tcttcggttc gttgttaacc 1440

tcggccgggc tttcctctct caagaacgca atgaagccga aagtgtacgt ggagaaaggc 1500

ggggtgcagg gtgttaggac cacaatcgaa aaactcaacc tcgatgggga tgaacttgcc 1560

cgtgctgtga gaaccgagct ctttgactta gataagacca caacaatcac cgttcagaaa 1620

aagtacaagt tattcgggcc taaagtcatc aatgtggata caacttatga cagcttcaag 1680

gctattagct ccgaggggat aaaaacaagt tcaggaaacg tgatgggcag aacgaggata 1740

ctggagttct cgaaccttga tgatgggctg aatgtaggca aattcacagt acgtgagctc 1800

gccaagatgg gagatgactc cgccaggctc ttctcagagg cacagaaaga catagtagcc 1860

tcaacggatg acatcctttc cttcgtggat gaggcaacct ccagtggaaa gagtgggcca 1920

acgaaaattg tgagaaccac cgactacatc acagatgagg gcttgaaaat aaagactctc 1980

caagagttct atgatgacat agtgaaaacc gagcagaaga ttacctcatt cgcaaaacca 2040

aggcccgtaa cagggtcgaa gaaggggagc tccttctcaa cgttagacga cctagtgaac 2100

aaagccgctt gtgatttgga cgaggtaata aagagcgtag ataccagaaa tcccatagga 2160

agtggtggag taacggtttt agaagaagac cccctcaagg ggcttactga cttggggttc 2220

aaggttagag aaaagccgac tacacggact gaggtgacac caaaatcttc ccccgtagcc 2280

ggcttcaaag ttccggatct ctccaccgag gtggaaaata tccttaatcc taatctctca 2340

gtgggagact ttgaacagga ccagaacaca ggttctacaa agcgctcccg tcgtagggga 2400

ggttcaaaga caatctccca cccagatatc agggtactca gcttgggaga acttgaagaa 2460

agtgtatttg gcataaacac ctcaccgagg gttagaattt caagtaagac aagttccggg 2520

cagagagatt accgcatccc cactcaagta gtcacaccaa aagaatttca catagatgag 2580

ccgaagtggg atatccggga cataacagac ccacttgagg acattgacag agaaacgcgg 2640

gcgattctct cacaggttct cgatacccgg ctcaccacca aaacaaaccc gaaaaaaccg 2700

aaatacaaga caaagaaaaa gaagaagagg ccgatacaaa cagcaccaaa gaagaagaaa 2760

agccaaaaga agagaaccaa aggcaagggt ggtgaatggg gaatctggat tggatacgaa 2820

agccttttgg actggtgat 2839

<210> 4
<211> 525
<212> DNA
<213> artificial

<220>
<223> Thermococcus nautilus 30/1 plasmid pTN2: ORF3 nucleic acid
      sequence

<400> 4
atgtctctaa aatcccgact tcgcgctttc cttggcctcc ctgaagtgga ggctgagatt 60

cgagaaataa aggagactct ctccaaactc tccgatgcac ttgaggacct ccaatccaaa 120

gtagatgctc aagatgaaca acttaagaga gccctcaccg agttgagccg caaggctgat 180

atgggattgt actacgagct aaatgaacgt ctcgaaacta cgagagatga aataaaggct 240

atggaagcgg aaataagctc aatctgggat aaggtgaagt ttatcgaatt ctcgttctcc 300

gagcattccg aagagcctga aatgaccgtt gaagagcttg caagcatcgt cgaacattac 360

attcgtaaag gtctaacccg cccatcagaa ctcaagaaag ctgttggggt ttcgtgggaa 420

aagctctatg cggcccttac ttacctcaaa ctaaagaaga gaatagcgag aattcaaaag 480

ggccgtagag tcgaatatgt tttagtggag gaagccgaag aataa 525

<210> 5
<211> 390
<212> DNA
<213> artificial

<220>
<223> Thermococcus nautilus 30/1 plasmid pTN2: ORF4 nucleic acid

sequence

<400> 5
atgacacgca agacatattt agggcacggt gggactatga ttgacagcat catcgaaaag 60

ctcgtagaaa tgggggcctt cccggcccgc aagaagacaa aaaccgagac catcagagtc 120

gtctcaaaca tgctcgaagc catcaaatac gaatacaacg gccggctacc acggtgggag 180

ctcaaaacct ttgtcacacg ctttgctaac caatcaaagt tctccgagcg acaaatctac 240

aggtacataa acgagctgaa agcattgggc tttctcgaag acatagtaga cgtaggaacc 300

cagaaacatt acatcgttct ctcaaagcgc tttggttccc ggatgcttgc cctttaccgg 360

gactacaaca aatggttagg ggaggtgtaa 390

<210> 6
<211> 295
<212> DNA
<213> artificial

<220>
<223> Thermococcus nautilus 30/1 plasmid pTN2: ORF5 nucleic acid
sequence

<400> 6
tggtgcggcc cttcaaagtt gtggtggttg gaaaaaccga gaatgagaag atatgggagt 60

tagaggccct cgtgtttgca aaggaccaaa ctcaagcaga agagcttagc ctcaccactg 120

ccaaaaaaag agggctccaa acccccaaag tggtgtacat gaaagaatac cgcagggcaa 180

gagtctacgc aatcggcaaa ttcggatggc ccgacgactg ggtatgggac ctcatagaag 240

aaggtcttgg ccaagctcca gccgggctcg tttcactaga ggacatagag gtgta 295

<210> 7
<211> 675
<212> DNA
<213> artificial

<220>
<223> Thermococcus nautilus 30/1 plasmid pTN2: ORF6 nucleic acid
sequence

<400> 7
atgagggact caagatattc ttcgtgggtc gagaaaaaac tacacgagga cctcacaaga 60

tattacaccc acagaacgct ggacgtctat gacatctggc tgattatgaa cctcctctat 120

gatggaacaa caatgcatag cctcgaaatc gagagagcag gagggaaaac ctacttcacg 180

ctagacaccc catccatctc atatggagtt tatgacccag agaagaggac cttcaccaca 240

aagtacgagc acattccagg accacacctg aacctaaaag aaatccacct caacatcccg 300

gaatccatag agaaactaaa agtgaaagga aaagagaaat ggctaatcac acgccgaaac 360

cccgtgaatt gggattatgc cccccgattc aagcctaaca tttacataca aggccgaatc 420

tggagcgatg acggagtaaa agggtggctt caacgaaaag aagatgagtt cgcctacgtc 480

gcaatcctcg gaacgatagt taccccctac gtattaggag aagacccaaa accggtattc 540

tatgaaagag aagtcttcat cgctcaaggg gcctttttgg gagacaccat ccaactcatc 600

cacaatttcc cgcacagccc caagataatc ccccgcagag tcattgaaca actccaacta 660

aaggaggggt tatga 675

<210> 8
<211> 519
<212> DNA
<213> artificial

<220>
<223> Thermococcus nautilus 30/1 plasmid pTN2: ORF7 nucleic acid
      sequence

<400> 8
atggcagatg ccgtgctttt agaaaatatc tacttcgccc taagccaagc cgacagggaa 60

acaaccgcaa gggggaaaat cgggtatctc ctaaccgccg cgtccctttt agaggccctc 120

gtcgaaaaag atagactaac aggcccaata acaattacaa aagtgaaata tgagctcgaa 180

caagacgaga ggggcaacta caaaagccgg agaccaagcg aagtggagag ggtaacactg 240

aatacaacag atgagataac ccttttcatt gaaaaacaca tcataagctc tctaatacaa 300

gttgcaagcc ttccccacac cgcaatgtta gacaaagtgt gggaattggg ctacaaaccc 360

gattgggggg aactacgcac aattcaaatt tttagctaca tcgaggaaat caaaagcctt 420

cttttgaaat tgctcgtcgc gctcgacctc gcgaacgtcg cattagtaga catagcaact 480

aagctagaga aatccacccg tagaaatacc actacataa 519

<210> 9
<211> 1107
<212> DNA
<213> artificial

<220>
<223> Thermococcus nautilus 30/1 plasmid pTN2: ORF8 nucleic acid
      sequence

<400> 9
gtgagtagcg tgaaaggcat gcaaatccac gcccgcaaag gagatgaact cctcgaaaag 60

ctactctcac aagaccccgc ttatggaggt agacaagccg ccatctacgg agccgttgga 120

agcggaaaga caaccctcct actctggctt gcccaagtct tacaagcccg cggagagtgg 180

ataatctgga gaggaagaga caccgacggc tggcacatgc tccacagctg gaagcaaaga 240

gtaaaactcc tcgtccacga gaaagacgaa gtccacgtct acaaagtccc ctacggaggc 300

accaccgcga ccgaaatcac gaagagtttg aacatcgaga agtacagtac cccagaggac 360

ctcctcacaa aacttgaaca agacaaaatc aacatcgtgt acgagcccac cttttaccag 420

ataacccaag agctcgccac ttacatcctc gccagaacca acatccggat accatcttgg 480

cagatgaaag aggccaaagg catttacttt tggtttgagc tcttcgagaa actcatcact 540

aggaaagacc gcagatggat aaccatcatc gtagacgagg ccgatgacct catcccagct 600

aacccccagg gcctccagtg gaggctacag gaatgggtaa agaactccgt caaggacttc 660

agaaagaacc tcatcaccct aattctcgcg gtccacaacg ccgaggactg cgaccaccgc 720

gtaatcagga agctccccat ccggatttac ttacagggag caagagtgga caaagacagc 780

ctgatgaccg acaaaacaat cacccaacgt ctcaaccccg gagaagcaaa aatcgaatgg 840

agaggcttcg gatacggagg cttcaaattc cctccactag aaaggcgcga ttacgccatt 900

atcgtcgaga agaaatggac cggagaactc cccaaattcg aagaagataa gcgaaccgca 960

gggagcatca tccaagaact ccgcgaaatc gccgaggaag aaggcatcga agaagccctc 1020

gaacgcgcat gggaactcgt ccaagaggga gtcatcacaa aacgctacta ctaccaaatc 1080

cgcaaagccc tccgcgaaga aagctga 1107

<210> 10
<211> 414
<212> DNA
<213> artificial

<220>
<223> Thermococcus nautilus 30/1 plasmid pTN2: ORF9 nucleic acid
 sequence

<400> 10
atgaatccct caattgagga aatcaaacgc cgtttgattc aagctggtgc atcgccacaa 60

gcagtcgaga gccttgaacc ggaattcttt gaggatctta ctgaagacat ggactttgag 120

gggactgcta gggttattaa tttcatagac tatctcgaaa actttgagaa ttacaagaga 180

aagcgagtga atattaccct tgctgtgccc gtgtatgagg ttctaaagca catcgcaagc 240

aagattgttg acgctgatgg tcgtccatac cccgtgagtt atcttatcga ggacataatt 300

gtttgggttc tgaaagatcc agataggttt gtgcaatttg tggaggaaac ttacccggag 360

gaggataatg atgagagcga ggagacacac tcggagattg aagagcaagc gtaa 414

<210> 11
<211> 180
<212> DNA
<213> artificial

<220>

<223>  Thermococcus nautilus 30/1 plasmid pTN2: ORF10 nucleic acid
       sequence

<400>  11
atgatgagag cgaggagaca cactcggaga ttgaagagca agcgtaaggt gggtgtagga      60

cgccctccag gacgaagaaa ggtgaatcac ctagatgtaa gcgctctcag cgcggagtgg     120

aaacagttgc tagatgtggt tgggcttggg agaaagaaaa gaaagggcct ttcatactga     180


<210>  12
<211>  489
<212>  DNA
<213>  artificial

<220>
<223>  Thermococcus nautilus 30/1 plasmid pTN2: ORF11 nucleic acid
       sequence

<400>  12
atgtccccca atactaagtg ctcggttccc cttaaaaaat caccgtatcc ttgtgagagt      60

gttaatgcag gagccacaat tcctagggat atctacacct tgctcgaaga acttagggcg     120

cttgacgttt tgaatgccaa gaattggaag gagatttacc tagctctctt gagaaatctt     180

acggagctta atgagcgttg gaagctcttg gcattaattt atcgcatcgt tgtatttcat     240

agcgacccct ccacggcaag tacggtcgtt ggcaagtttc tcaagaattt tgacattcta     300

tttgaaaaac tccccggcca ccgggcttat ttcgccccca ctcaggtggg gatgtggctt     360

tttgagaatc ttatcaaaca gggagggcag ggagagatag agttcagggc tgagcgccaa     420

acgtcagttg tgcagcagct atccttaatg tccttcctcc agaacacccc taagggggtg     480

aaagtatga                                                             489


<210>  13
<211>  2772
<212>  DNA
<213>  artificial

<220>
<223>  Thermococcus nautilus 30/1 plasmid pTN2: ORF12 (tn2-12p) nucleic
       acid sequence

<400>  13
   atgagcagtc ttcgtccatc ttcaataatc attgatatct acaaggttat tcaggatcac      60
   ccagattcca gccggtgggc tattgaactt cgcttccttc cgaagccgat atcttcggag     120
   tggattttca ttactgacat tgaggaaaga gcttctgaga ttgataaggt tcttactaag     180
   tacaacataa tgaagaagaa agacgcctac gtctctatgg cgatacacga tttcgagaag     240
   gttaccgcga agctgaagcg tgtccaagaa gaagagaaca aaaaagccac ggagggagag     300
   aggaggctta ggaggatcac cctagacaaa atccaggggg attcagaaac cacctcaggt     360
   ttcactctcg ctcttgtggt tgatattgac aataccaaga tccacgacac taggattatt 420
   gagaatgagg aggaggcctt tgaggcatca aaaagagagt gggaggcgct taagcctaaa 480
   cttcaagaac tggggttcct cccaagatgg attctttaca caggcggagg gcttcagctc 540
   tggtttgtca gtgacaaact cgaacccatc tcagtgattg acagggcctc ggagattatc 600
   ccgaacatta tgaatggggt gaatggcgtc aaggggcttc tttccgaggg gttcaaggct 660
   gacaacatct ttgatcctgc taggatagtt agggcgccgc tcactttcaa tcataagtac 720
   aggaccatca taaaggacga agacggaacg gagagggttg ttcccacaca ggtaaaaggt 780
   cgcgttattg agtttaacga cgtcaggatt tcgctcacgg agttccttga caggctggag 840
   gcctacgcta aagagaaagg gattcctctc gaaaagccca ctaagcggaa gttccttgaa 900
   ctggcttcaa agagatacga agtcacgtcc tcgaactttg aagccctcgc cgagagactc 960
   ttcacggagc ttaggccttg gtgggagata gccaaggaaa agggctggtc taggcaccat 1020
   ctcacgatgg gcatagctac ttacatcttg aggaacacta atctaacccc ggagcagctc 1080
   attggaagtg aaaactcacc ggggctttgg gagctcgtct tcgccaaact tgttgaagca 1140
   ggactggaag atccggatga ttggaagaac agggcctcca cgataaggga cgcctacaag 1200
   aagatagaga gtggaaagaa ggtagcaacg aaggcctacc tccggaagta cattgagggc 1260
   ctttcggagg aggatgctgt ccagattctc ctctctgtga agagggcact cctcccttac 1320
   ctaaaagcgg tggatgtgaa gaggatctct aagtacagcg caaggccata tgagattaca 1380
   gaagaaatcc caaagagctg ggaagatatt gacgagaatc gtaaaaaagc aactggagtt 1440
   tggtacatag atttccttgc tctcgaaaca gcgaatgaca tttacttcga ggatcttcct 1500
   aagcctcccg ttttctacat tcggtttgtt gaaaagaaca aggagaaatt taagcttaat 1560
   gaaactctct atcattcctt tctgacttgg ctggggatca ccgaaggaga gccactcgat 1620
   agaactgagc tgattgacct tctcgtggag aaatttggtt ttactgttga ggacctcaag 1680
   gctatctact acagaaagat cctcaccctt ctaaaacctg aggggatgag gacccccaag 1740
   tgcatccagg agttcctctt tgagctcgcc acggaaggta atctcccaga agataagatt 1800
   cgacacttgg cacattgggt taagttctat gctagacccc ttcggcattc gaccacgtca 1860
   gtactcctta aaggcagagg caagccagta gatatgaggc tggcaatttg ggccaaagtc 1920
   gttgagttct tcgccgaaga tgatgaggta gctgaggagt taataactac cttcaagaaa 1980
   gcatacatgg gagctgagcc tcccttcccc tgcatcggag ctgagtcatg cccattctat 2040
   cctgaccaca gggcttgtcc gttcatagtt ccaaagcgca aagaggttct accagtgagc 2100
   attgtggacg ttcagttaca cggctcggat ggcattgttg ttttagtcgg tggtccgaca 2160
   gaggtaacag cgtttactct cgaaggaaaa gtcgagtgga taaagaccac taaaaagaca   2220
   gtcaagtatc ccatagccga gtggttcctt gacaggtttg ccaaagagta cttgtctctc   2280
   ccggaggctc cttcttggtg gaagcttgag gaggttactg agatactgaa aagtcgggcg   2340
   agggttgtta aaagccaatt tgacaagttt gaggactacc ttgaacagtt catcgagtgg   2400
   ctccagaagg agaattcccg ccgggggata ctcccctacg aaaaggccga tgagaaccac   2460
   ttattcatca agggcgaatg ggtagggatc ccgccaggat ttgctaggga attttacagt   2520
   ggcgaacttc tcataggtgg cccaaccttc aggagaatgc tcgaacaaaa gctcggaaag   2580
   gattaccgga agatgtcggc aaaaatccac ctcaacacgg gcctaaagga caagaggaac   2640
   tgctacttcg tgagtgtgga gtggttcaga aagcatgtag gcgaacccaa catccaagag   2700
   ataacctccg agggggatgt ctcatttgac ggtctcagct atgacgatga agaggagggg   2760
   gtggtcgggt ga                                                         2772

<210>  14
<211>  923
<212>  PRT
<213>  artificial

<220>
<223>  Thermococcus nautilus 30/1 plasmid pTN2: tn2-12p protein sequence

<400>  14

Met Ser Ser Leu Arg Pro Ser Ser Ile Ile Ile Asp Ile Tyr Lys Val
1               5                   10                  15

Ile Gln Asp His Pro Asp Ser Ser Arg Trp Ala Ile Glu Leu Arg Phe
            20                  25                  30

Leu Pro Lys Pro Ile Ser Ser Glu Trp Ile Phe Ile Thr Asp Ile Glu
        35                  40                  45

Glu Arg Ala Ser Glu Ile Asp Lys Val Leu Thr Lys Tyr Asn Ile Met
    50                  55                  60

Lys Lys Lys Asp Ala Tyr Val Ser Met Ala Ile His Asp Phe Glu Lys
65                  70                  75                  80

Val Thr Ala Lys Leu Lys Arg Val Gln Glu Glu Glu Asn Lys Lys Ala
                85                  90                  95

Thr Glu Gly Glu Arg Arg Leu Arg Arg Ile Thr Leu Asp Lys Ile Gln
            100                 105                 110

Gly Asp Ser Glu Thr Thr Ser Gly Phe Thr Leu Ala Leu Val Val Asp
        115                 120                 125

Ile Asp Asn Thr Lys Ile His Asp Thr Arg Ile Ile Glu Asn Glu Glu
    130                 135                 140

Glu Ala Phe Glu Ala Ser Lys Arg Glu Trp Glu Ala Leu Lys Pro Lys

145                 150                 155                 160

Leu Gln Glu Leu Gly Phe Leu Pro Arg Trp Ile Leu Tyr Thr Gly Gly
                165                 170                 175

Gly Leu Gln Leu Trp Phe Val Ser Asp Lys Leu Glu Pro Ile Ser Val
            180                 185                 190

Ile Asp Arg Ala Ser Glu Ile Ile Pro Asn Ile Met Asn Gly Val Asn
        195                 200                 205

Gly Val Lys Gly Leu Leu Ser Glu Gly Phe Lys Ala Asp Asn Ile Phe
    210                 215                 220

Asp Pro Ala Arg Ile Val Arg Ala Pro Leu Thr Phe Asn His Lys Tyr
225                 230                 235                 240

Arg Thr Ile Ile Lys Asp Glu Asp Gly Thr Glu Arg Val Val Pro Thr
                245                 250                 255

Gln Val Lys Gly Arg Val Ile Glu Phe Asn Asp Val Arg Ile Ser Leu
            260                 265                 270

Thr Glu Phe Leu Asp Arg Leu Glu Ala Tyr Ala Lys Glu Lys Gly Ile
        275                 280                 285

Pro Leu Glu Lys Pro Thr Lys Arg Lys Phe Leu Glu Leu Ala Ser Lys
    290                 295                 300

Arg Tyr Glu Val Thr Ser Ser Asn Phe Glu Ala Leu Ala Glu Arg Leu
305                 310                 315                 320

Phe Thr Glu Leu Arg Pro Trp Trp Glu Ile Ala Lys Glu Lys Gly Trp
                325                 330                 335

Ser Arg His His Leu Thr Met Gly Ile Ala Thr Tyr Ile Leu Arg Asn
            340                 345                 350

Thr Asn Leu Thr Pro Glu Gln Leu Ile Gly Ser Glu Asn Ser Pro Gly
        355                 360                 365

Leu Trp Glu Leu Val Phe Ala Lys Leu Val Glu Ala Gly Leu Glu Asp
    370                 375                 380

Pro Asp Asp Trp Lys Asn Arg Ala Ser Thr Ile Arg Asp Ala Tyr Lys
385                 390                 395                 400

Lys Ile Glu Ser Gly Lys Lys Val Ala Thr Lys Ala Tyr Leu Arg Lys
                405                 410                 415

Tyr Ile Glu Gly Leu Ser Glu Glu Asp Ala Val Gln Ile Leu Leu Ser
            420                 425                 430

Val Lys Arg Ala Leu Leu Pro Tyr Leu Lys Ala Val Asp Val Lys Arg
        435                 440                 445

Ile Ser Lys Tyr Ser Ala Arg Pro Tyr Glu Ile Thr Glu Glu Ile Pro
    450                 455                 460

Lys Ser Trp Glu Asp Ile Asp Glu Asn Arg Lys Lys Ala Thr Gly Val
465                 470                 475                 480

Trp Tyr Ile Asp Phe Leu Ala Leu Glu Thr Ala Asn Asp Ile Tyr Phe
                485                 490                 495

Glu Asp Leu Pro Lys Pro Pro Val Phe Tyr Ile Arg Phe Val Glu Lys
            500                 505                 510

Asn Lys Glu Lys Phe Lys Leu Asn Glu Thr Leu Tyr His Ser Phe Leu
        515                 520                 525

Thr Trp Leu Gly Ile Thr Glu Gly Glu Pro Leu Asp Arg Thr Glu Leu
    530                 535                 540

Ile Asp Leu Leu Val Glu Lys Phe Gly Phe Thr Val Glu Asp Leu Lys
545                 550                 555                 560

Ala Ile Tyr Tyr Arg Lys Ile Leu Thr Leu Leu Lys Pro Glu Gly Met
                565                 570                 575

Arg Thr Pro Lys Cys Ile Gln Glu Phe Leu Phe Glu Leu Ala Thr Glu
            580                 585                 590

Gly Asn Leu Pro Glu Asp Lys Ile Arg His Leu Ala His Trp Val Lys
        595                 600                 605

Phe Tyr Ala Arg Pro Leu Arg His Ser Thr Thr Ser Val Leu Leu Lys
    610                 615                 620

Gly Arg Gly Lys Pro Val Asp Met Arg Leu Ala Ile Trp Ala Lys Val
625                 630                 635                 640

Val Glu Phe Phe Ala Glu Asp Asp Glu Val Ala Glu Glu Leu Ile Thr
                645                 650                 655

Thr Phe Lys Lys Ala Tyr Met Gly Ala Glu Pro Pro Phe Pro Cys Ile
            660                 665                 670

Gly Ala Glu Ser Cys Pro Phe Tyr Pro Asp His Arg Ala Cys Pro Phe
        675                 680                 685

Ile Val Pro Lys Arg Lys Glu Val Leu Pro Val Ser Ile Val Asp Val
    690                 695                 700

Gln Leu His Gly Ser Asp Gly Ile Val Val Leu Val Gly Gly Pro Thr
705                 710                 715                 720

Glu Val Thr Ala Phe Thr Leu Glu Gly Lys Val Glu Trp Ile Lys Thr
                725                 730                 735

Thr Lys Lys Thr Val Lys Tyr Pro Ile Ala Glu Trp Phe Leu Asp Arg
            740                 745                 750

Phe Ala Lys Glu Tyr Leu Ser Leu Pro Glu Ala Pro Ser Trp Trp Lys
        755                 760                 765

Leu Glu Glu Val Thr Glu Ile Leu Lys Ser Arg Ala Arg Val Val Lys
    770                 775                 780

Ser Gln Phe Asp Lys Phe Glu Asp Tyr Leu Glu Gln Phe Ile Glu Trp
785                 790                 795                 800

Leu Gln Lys Glu Asn Ser Arg Arg Gly Ile Leu Pro Tyr Glu Lys Ala
                805                 810                 815

Asp Glu Asn His Leu Phe Ile Lys Gly Glu Trp Val Gly Ile Pro Pro
            820                 825                 830

Gly Phe Ala Arg Glu Phe Tyr Ser Gly Glu Leu Leu Ile Gly Gly Pro
        835                 840                 845

Thr Phe Arg Arg Met Leu Glu Gln Lys Leu Gly Lys Asp Tyr Arg Lys
    850                 855                 860

Met Ser Ala Lys Ile His Leu Asn Thr Gly Leu Lys Asp Lys Arg Asn
865                 870                 875                 880

Cys Tyr Phe Val Ser Val Glu Trp Phe Arg Lys His Val Gly Glu Pro
                885                 890                 895

Asn Ile Gln Glu Ile Thr Ser Glu Gly Asp Val Ser Phe Asp Gly Leu
            900                 905                 910

Ser Tyr Asp Asp Glu Glu Glu Gly Val Val Gly
        915                 920


<210>  15
<211>  20
<212>  DNA
<213>  artificial

<220>
<223>  20 nt oligonucleotide short primer-template substrate

<400>  15
cgaacccgtt ctcggagcac                                                   20


<210>  16
<211>  42
<212>  DNA
<213>  artificial

<220>
<223>  42 nt oligonucleotide template

<400>  16
ttctgcacaa agcggttctg cagtgctccg agaacgggtt cg                          42


<210>  17
<211>  18
<212>  DNA
<213>  artificial

<220>
<223>  18 nt oligonucleotide short primer-template substrate

<400>  17
gtaaaacgac ggccagtg                                                     18

<210>  18
<211>  19
<212>  DNA
<213>  artificial

<220>
<223>  RBS and start codon ORF1

<400>  18
aggaggggt ggtcgggtg                                                    19


<210>  19
<211>  15
<212>  DNA
<213>  artificial

<220>
<223>  RBS and start codon ORF2

<400>  19
   gggggatttt gtatg                                                        15


<210>  20
<211>  15
<212>  DNA
<213>  artificial

<220>
<223>  RBS and start codon ORF3

<400>  20
tgggggatga cgatg                                                       15


<210>  21
<211>  19
<212>  DNA
<213>  artificial

<220>
<223>  RBS and start codon ORF4

<400>  21
gggtggtgtc gtaattatg                                                   19


<210>  22
<211>  19
<212>  DNA
<213>  artificial

<220>
<223>  RBS and start codon ORF5

<400>  22
aggggaggtg taaccgatg                                                   19

<210>  23
<211>  18
<212>  DNA
<213>  artificial

<220>
<223>  RBS and start codon ORF6

<400>  23
agaggtgtaa aacaaatg                                                    18

<210>  24
<211>  16
<212>  DNA
<213>  artificial

<220>
<223>  RBS and start codon ORF7

<400>  24
aggaggggtt atgatg                                                      16

<210>  25
<211>  17
<212>  DNA
<213>  artificial

<220>
<223>  RBS and start codon ORF8

<400>  25
tggaggtgcc gagcgtg                                                     17

<210>  26
<211>  17
<212>  DNA
<213>  artificial

<220>
<223>  RBS and start codon ORF9

<400>  26
agggggtact cataatg                                                     17

<210>  27
<211>  15
<212>  DNA
<213>  artificial

<220>
<223>  RBS and start codon ORF10

<400>  27
cggaggagga taatg                                                       15

<210>  28
<211>  17
<212>  DNA
<213>  artificial

<220>
<223>  RBS and start codon ORF11

<400>  28
tggaggtgat gtaaatg                                                    17


<210>  29
<211>  16
<212>  DNA
<213>  artificial

<220>
<223>  RBS and start codon ORF12

<400>  29
   agggggtgaa agtatg                                                      16

## Description

The present invention is directed to a thermostable primase/DNA polymerase protein of the *Thermococcus nautilus* 30/1 plasmid pTN2 and the nucleic acid encoding said primase/DNA polymerase. The invention also relates to method of synthesizing, amplifying or sequencing nucleic acid implementing said primase/DNA polymerase protein and kit comprising said DNA polymerase protein.

Plasmids are present in many archaeal species including archaeons living in geothermally heated aquatic environments (Lipps et al., 2008). Their size range from a very small plasmid with a single gene, the plasmid pRQ7 from the hyperthermophilic bacterium Thermotoga (Harriott et al., 1994), to large megaplasmids whose size rival those of bacterial or archaeal chromosomes (Baliga et al., 2004; Soppa, 2006). They can use different replication strategies (mostly rolling-circle mode for the smallest ones and theta mode for the others) and a variety of replication origins (del Solar et al., 1998).

In Archaea, plasmids from Sulfolobales (thermoacidophilic members of the phylum Crenarchaea) have been especially well characterized. Two plasmid families have been identified: the pRN plasmids and relatives (with sizes ranging mainly from 5 to 8 kb, up to 13.6 kb), and a family of rather large conjugative plasmids (CP), the pNOB8 and relatives (with sizes ranging from 24 to 36 kb) (Lipps, 2006). The pRN plasmids harbour three genes which are also more or less conserved in pRN relatives. Two of them encode site-specific DNA binding proteins (CopG and PlrA) that could be involved in regulation of copy number and gene expression (Lipps et al., 2001a; Lipps et al., 2001b). The third gene encodes a large protein, RepA, involved in plasmid replication. The RepA protein of the plasmid pRN1 is a multifunctional enzyme whose N-terminal domain harbours DNA primase/polymerase activities and the C-terminal domain harbours a DNA helicase activity (Lipps, 2004; Lipps et al., 2003). The structural characterization of the N-terminal domain led to the identification of a new family of DNA polymerase (family E) distantly related to the archaeal/eukaryal DNA primase catalytic domain (Lipps et al., 2004). The larger genome of the conjugative plasmids (CP) from Sulfolobus species encodes around 40-50 proteins each (Erauso et al., 2006; Greve et al., 2004). Ten proteins are conserved in all CP plasmids of Sulfolobales and 80% of the others are common to two or more CP plasmids (Erauso et al., 2006). Most of these proteins are of unknown function.All plasmids isolated up to now from Sulfolobus species are larger than 5kb. Although their mode of replication has not yet been determined experimentally, they probably replicate via the theta mode, since none of them encode the Rep protein typical for rolling-circle replication. In contrast, all plasmids isolated up to now from Thermococcales (hyperthermophilic and anaerobic members of the phylum Euryarhaea) are small (less than 4 kb) and seems to replicate via the rolling circle (RC) mode. This has been experimentally demonstrated for the plasmids pGT5 from *Pyrococcus abyssi* (Erauso et al., 1996; Marsin & Forterre, 1998; Marsin & Forterre, 2001) and is most likely for the related plasmid pTN1, from the candidate species *Thermococcus nautilus* (Soler et al., 2007). Both plasmids encode large homologous proteins related to Rep proteins known to initiate rolling circle replication. A third small plasmid, called pRT1, from Pyrococcus sp. JT1, has been sequenced and tentatively described as a RC plasmid (Ward et al., 2002). However, its putative Rep protein shows no clear sequence similarity with known RC Rep proteins.

A variety of nucleic acid amplification techniques, developed as tools for nucleic acid analysis and manipulation, have been successfully applied for clinical diagnosis of genetic and infectious diseases. Amplification techniques can be grouped into those requiring temperature cycling (PCR and ligase chain reaction) and isothermal systems (amplification systems (3SR and NASBA), strand-displacement amplification, and Qβ replication systems). Two aspects are frequent caveats in these procedures: fidelity of synthesis and length of the amplified product. Development of an amplification system relying on the mechanism of DNA replication using such primase/DNA polymerase has been the object of many publications and patent documents (see for example Dean et al., Genome Res. 2001 Jun;11(6):1095-9; Mendez et al., EMBO J., 1997, 1;16(9):2519-27; Hutchison et al., Proc Natl Acad Sci U S A., 2005, 102(48):17332-6; Mamone, Innovations Forum: GenomiPhi DNA amplification, Life Sciences News 14, 2003 Amersham Biosciences; Blanco et al., 1994; EP 0 862 656 or U.S. 5,001,050).

Currently there is a need for a new thermostable highly processive DNA polymerase belonging to the protein-primed DNA polymerase family which can work at high temperature and which is able to produce long extension products (more than one Kb in length, preferably more than 5 Kb) without the need of the presence of additional proteins.

There is particularly a great interest to provide with a such thermostable DNA polymerase, soluble in aqueous solvent and easy to produce by recombinant method, preferably in *E. coli* bacteria which is well known by the skilled person and which is easy to cultivate.

Particularly waited are such soluble thermostable DNA polymerase easy to produce in *E. coli* which exhibits a primase activity and which is consequently able to synthesize or amplify unknown DNA template, preferably in total absence of primer and in addition which is able to produce with fidelity long extension products (more than one Kb in length, preferably more than 5 Kb or 10 Kb) without the need of the presence of additional proteins.

The activity of a nucleotidyl transferase, an important cellular enzyme involved in the DNA processing and repair, is also advantageous for the cloning purposes. For example this activity is a characteristic property of Taq polymerase. Taq polymerase makes DNA products that have A (adenine) overhangs at their 3' ends. This may be useful in TA cloning, whereby a cloning vector (such as a plasmid) is used which has a T (thymine) 3' overhang, which complements with the A overhang of the PCR product, thus enabling ligation of the PCR product into the plasmid vector.

This is the object of the present invention.

The inventors have characterized the plasmid pTN1 and demonstrated that the strain *T. nautilus* contains additionally a larger plasmid of around 13 kb, which has been called pTN2. After sequencing and annotation of the complete sequence of the *Thermococcus nautilus* 30/1 plasmid pTN2 (13 kb, Sequence SEQ ID NO: 1), the inventors have demonstrated the presence of a nucleic sequence ORF12 (SEQ ID NO: 13) that encodes a protein of 107 kDa (tn2-12 having the sequence SEQ ID NO: 14). A BLAST search did not allow to predict the function of this protein. However, by using PSI-BLAST iterations, the inventors have retrieved many hits between their N terminal regions and the same regions of proteins annotated either as uncharacterized proteins or as putative Rep protein or DNA primases. Interestingly, it has been demonstrated that all these N-terminal regions exhibit a conserved DhD motif, known to be present in the active sites of many DNA polymerases, primases and/or nucleotidyl transferases. Interestingly, using PSI-BLAST searches, the inventors have also demonstrated that the middle part of tn2-12 is composed of a short stretch of highly conserved amino-acids found in many proteins with the S1 RNA binding motif usually detected in proteins able to bind nucleic acids.

After testing the DNA polymerase activity of tn2-12, the inventors have demonstrated that the protein tn2-12 is able to extend the primer up to several kilobases in less than 20 min of the reaction time with a highest activity found between 70°C and 80°C, and that the obtained DNA shows the same pattern as that synthesized by the Taq polymerase. Additionally to the DNA polymerase activity, the inventors have demonstrated that tn2-12 has a clear primase activity, the protein being able to efficiently initiate the second strand synthesis in the presence of ssDNA template.

These results confirm that tn2-12 is a thermostable primase/processive DNA polymerase able to produce long extension products, its activity being independent of the presence of additional proteins.

As any previously known DNA polymerases were detected by BLAST search, it can be considered that this discovered enzyme belongs to a new family of the DNA depending DNA polymerases.

So, in a first aspect, the present invention is directed to an isolated polypeptide, wherein:
a) the sequence of said polypeptide comprises the three following motifs:
   Motif I: DhD;
   Motif II: S/TG-GhQ/H; and
   Motif III: D---D--RhhRhP---N, and
b) said polypeptide harbours DNA polymerase and primase activities.

In these motifs, a dash ("-") or "h" designates any amino acid residue. The letters D, S, T, G, Q, H, R; P and N designate the specific single-letter amino acid code.

The linear order in which these three motifs are present in the sequences of the isolated polypeptides is indifferent.

In a preferred embodiment said polypeptide harbours further nucleotidyl transferase activity in addition to said DNA polymerase and primase activities.

In a preferred embodiment these three motifs are presented in a linear order I-II-III beginning from the N-terminal end of the polypeptide.

Deoxyribonucleic acid (DNA) polymerase, primase, nucleotidyl transferase or reverse transcriptase enzymatic activity is standard activity well known by the skilled person.

For example, by DNA polymerase activity, it can be intended to designate the activity of an enzyme that catalyzes the polymerization of deoxyribonucleotides into a DNA strand. DNA polymerases enzymes are best-known for their role in DNA replication, in which the polymerase "reads" an intact DNA strand as a template and uses it to synthesize the new strand. This process copies a piece of DNA. The newly-polymerized molecule is complementary to the template strand and identical to the template's original partner strand.

The term "DNA polymerase activity" refers for example to the ability of an enzymatic polypeptide to synthesize new DNA strands by the incorporation of deoxynucleoside triphosphates. The example 4 below provides an example of assay for the measurement of DNA polymerase activity. Such DNA polymerase activity may be measured using any of the DNA polymerase activity assays well known by the skilled person. A protein which can direct the synthesis of new DNA strands (DNA synthesis) by the incorporation of deoxynucleoside triphosphates in a template-dependent manner is said to be "capable of DNA polymerase activity".

For example, by DNA primase activity, it can be intended to designate the activity of an enzyme which is involved in the initiation of DNA replication, said enzyme catalyzes the polymerization of short ribonucleic acid (RNA) primers on the template DNA.

For example, by nucleotidyl transferase activity, it can be intended to designate the catalysis of the transfer of a nucleotidyl group to a reactant. In mammalians the corresponding enzyme is involved in the non-homologous end joining (NHEJ) process that is responsible for repairing double strand breakes in the DNA (Paull, 2005). For example, the nucleotidyl transferase activity of Taq polymerase is widely used in the T/A cloning system.

In a preferred embodiment, the polypeptide according to the present invention has been isolated from hyperthermophilic Archaea or Bacteria.

In a preferred embodiment, the polypeptide according to the present invention exhibits a DNA polymerase activity at a temperature of superior or equal to 65°C, more preferably superior or equal to 70°C, still more preferably comprised between 70°C and 80°C.

By thermophilic Archaea or Bacteria, it is intended to designate a member of Archaea or Bacteria that is capable of best growth in temperatures comprised between 45°C and 100°C and more.
Thermophiles are classified into:
- moderate thermophiles which are intended to designate members that are capable of best growth in temperatures comprised between 45°C and 65°C;
- extreme thermophiles which are intended to designate members that are capable of best growth in temperatures comprised between 65°C and 80°C; and
- hyperthermophiles which are intended to designate members that are capable of best growth in temperatures superior to 80°C,
(members that are capable of best growth in temperatures comprised between 25°C and 45°C being designated by mesophile and members that are capable of best growth in temperatures comprised between -5°C and 25°C being designated by psychrophile).

In a more preferred embodiment, the polypeptide according to the present invention has been isolated from a member of the phylum Euryarhaea.

In a preferred embodiment, the polypeptide according to the invention or the nucleic acid encoding thereof is isolated from a *Thermococcus sp,* or plasmid or virus thereof, preferably from a *Thermococcus sp* plasmid or virus, more preferably from a *Thermococcus nautilus* variants plasmid or from a pTN2 plasmid variant of *Thermococcus nautilus,* more preferably from the gene ORF12 having the sequence SEQ ID NO: 13, or variant thereof, encoding said DNA polymerase.

By variant of an archaea or a bacteria, virus, plasmid or gene, it is intended to designate in the present invention a natural variant of said archaea, bacteria, virus, plasmid or gene which can be isolated, or a variant of the wild type archaea, bacteria, virus, plasmid or gene which has been obtained by mutation, for example by a method implementing a site-directed mutagenesis step.

In a more preferred embodiment, the polypeptide according to the present invention is encoded by a nucleic acid isolated from a pTN2 plasmid having the sequence SEQ ID NO: 1 or a variant thereof.

By a variant of a pTN2 plasmid, it is intended to designate a plasmid having a nucleic sequence exhibiting at least 70 % identity, preferably 75 %, 80 %, 85 %, 90 %, 92 %, 95 %, 97,5 %, 98 %, 98,5 %, 99 % and 99,5 % identity, with the sequence SEQ ID NO: 1 depicting the nucleic acid sequence of the *Thermococcus nautilus* 30/1 plasmid pTN2.

Among the variants of a pTN2 plasmid, the variant exhibiting an ORF sequence having at least 70 % identity, preferably 75 %, 80 %, 85 %, 90 %, 92 %, 95 %, 97,5 %, 98 %, 98,5 %, 99 % and 99,5 % identity, with the sequence SEQ ID NO: 13 are preferred.

In a preferred embodiment, the polypeptide according to the present invention further exhibits a reverse transcriptase activity.

For example by reverse transcriptase activity, it can be intended to designate the catalysis of RNA-template-directed extension of the 3'- end of a DNA strand by one deoxynucleotide at a time.

The present invention is also directed to an isolated polypeptide or to an isolated polypeptide according to the present invention, wherein said polypeptide comprises a polypeptide selected from the group of polypeptides consisting of:
a) the polypeptide having the amino acid sequence SEQ ID NO: 14;
b) a fragment of a) having a DNA polymerase activity or/and a DNA primase activity and/or a nucleotidyl transferase activity;
c) a polypeptide having sequence which is at least 70 % identity, preferably 75 %, 80 %, 85 %, 90 %, 92 %, 95 %, 97,5 %, 98 %, 98,5 %, 99 % and 99,5 %, after optimum alignment with the sequence SEQ ID NO: 14, or with a sequence as defined in b), said polypeptide having a DNA polymerase activity and/or DNA primase activity and/or nucleotidyl transferase activity, preferably a DNA polymerase activity at a temperature of superior or equal to 65°C, more preferably superior or equal to 70°C, still more preferably comprised between 70°C and 80°C.

In a preferred embodiment, said polypeptide according to the present invention or one of their fragments is a polypeptide having DNA polymerase and primase and transferase activities.

In a preferred embodiment, said polypeptide according to the present invention or one of their fragments is a polypeptide further exhibiting a reverse transcriptase activity.

In a preferred embodiment, said fragment having a DNA polymerase and/or primase activity and/or nucleotidyl transferase activity, has at least 50, 100, 150, 200, 250, 300, 350, 400, 450, 500 or 550 amino acids.

In a preferred embodiment, said polypeptide according to the present invention or one of their fragments is a polypeptide further exhibiting a reverse transcriptase activity.

In a preferred embodiment, the DNA polymerase according to the invention or the nucleic acid encoding thereof is isolated from a *Thermococcus sp,* or plasmid or virus thereof, preferably from a *Thermococcus sp* plasmid or virus, from a *Thermococcus nautilus* variants plasmid or from a pTN2 plasmid variant of *Thermococcus nautilus,* more preferably from the gene, or variant thereof, encoding said DNA polymerase.

By variant of a bacteria, virus, plasmid or gene, it is intended to designate in the present invention a natural variant of said bacteria, virus, plasmid or gene which can be isolated, or a variant of the wild type bacteria, virus, plasmid or gene which has been obtained by mutation, for example by a method implementing a site-directed mutagenesis step.

In a more preferred embodiment, the polypeptide of the present invention, or fragments thereof, comprises at least one the three following motifs:
DhD;
S/TG-GhQ/H; or
D---D--RhhRhP---N,
preferably two of these three motifs, more preferably the three motifs.

The term "DNA polymerase activity" refers to the ability of an enzymatic polypeptide to synthesize new DNA strands by the incorporation of deoxynucleoside triphosphates. The example 4 below provides an example of assay for the measurement of DNA polymerase activity. Such DNA polymerase activity may be measured using any of the DNA polymerase activity assays well known by the skilled person. A protein which can direct the synthesis of new DNA strands (DNA synthesis) by the incorporation of deoxynucleoside triphosphates in a template-dependent manner is said to be "capable of DNA polymerase activity".

In the present description, the terms polypeptides, polypeptide sequences, peptides and proteins are interchangeable.

In the present description, the terms nucleic acid, polynucleotide, oligonucleotide, or acid nucleic or nucleotide sequence are interchangeable.

The terms "identical" or percent "identity", in the context of two or more polypeptide sequences, refer to two or more sequences or subsequences that are the same or have a specified percentage of amino acid residues that are the same (i.e., about 70 % identity, preferably 75 %, 80 %, 85 %, 90 %, 92 %, 95 %, 97,5 %, 98 %, 98,5 %, 99 % and 99,5 %, or higher identity over a specified region when compared and aligned for maximum correspondence over a comparison window or designated region) as measured using a BLAST or BLAST 2.0 sequence comparison algorithms with default parameters, or by manual alignment and visual inspection (see, e.g., NCBI web site). The definition also includes sequences that have deletions and/or additions, as well as those that have substitutions. As described below, the preferred algorithms can account for gaps and the like. Preferably, identity exists over a region that is at least about 25 amino acids in length, or more preferably over a region that is 25-75 amino acids in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

Methods of alignment of sequences for comparison are well-known in the art. A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990).

For example, it is possible to use the BLAST program, "BLAST 2 sequences" (Tatusova et al., "Blast 2 sequences - a new tool for comparing protein and nucleotide sequences", FEMS Microbiol Lett. 174:247-250, 1999) available on the site http://www.ncbi.nlm.nih.gov/gorf/b12.html, the parameters used being those given by default (in particular for the parameters "open gap penalty": 5, and "extension gap penalty": 2; the matrix chosen being, for example, the matrix "BLOSUM 62" proposed by the program), the percentage of identity between the two sequences to be compared being calculated directly by the program.

By amino acid sequence having at least 70 % identity, preferably 75 %, 80 %, 85 %, 90 %, 92 %, 95 %, 97,5 %, 98 %, 98,5 %, 99 % and 99,5 %, or higher identity with a reference amino acid sequence, those having, with respect to the reference sequence, certain modifications, in particular a deletion, addition or substitution of at least one amino acid, a truncation or an elongation are preferred. In the case of a substitution of one or more consecutive or non consecutive amino acid(s), the substitutions are preferred in which the substituted amino acids are replaced by "equivalent" amino acids. The expression "equivalent amino acids" is aimed here at indicating any amino acid capable of being substituted with one of the amino acids of the base structure without, however, essentially modifying the DNA polymerase activity of the reference polypeptide and such as will be defined later, especially in the example 4, last paragraph.

These equivalent amino acids can be determined either by relying on their structural homology with the amino acids which they replace, or on results of comparative trials of DNA polymerase activity between the different polypeptides capable of being carried out. By way of example, mention is made of the possibilities of substitution capable of being carried out without resulting in a profound modification of the DNA polymerase activity of the corresponding modified polypeptide. It is thus possible to replace leucine by valine or isoleucine, aspartic acid by glutamic acid, glutamine by asparagine, arginine by lysine, etc., the reverse substitutions being naturally envisageable under the same conditions.

In a second aspect, the present invention provides a nucleic acid encoding a polypeptide having a DNA polymerase and/or primase activity and/or nucleotidyl transferase activity according to the invention, particularly the nucleic acid having the sequence SEQ ID NO: 13 or having a sequence which is at least 70 % identity, preferably 75 %, 80 %, 85 %, 90 %, 92 %, 95 %, 97,5 %, 98 %, 98,5 %, 99 % and 99,5 %, or higher identity after optimum alignment with the sequence SEQ ID NO: 13, the polypeptide encoded by said nucleic acid having a DNA polymerase activity, preferably at a temperature of superior or equal to 65°C, preferably superior or equal to 70°C, more preferably comprises between 70°C and 80°C.

In another aspect, the invention encompasses a vector, preferably a cloning or an expression vector, comprising the nucleic acid of the invention.

In a preferred embodiment, the vector according to the invention is characterized in that said nucleic acid is operably linked to a promoter.

The invention aims especially at cloning and/or expression vectors which contain a nucleotide sequence according to the invention.

The vectors according to the invention preferably contain elements which allow the expression and/or the secretion of the nucleotide sequences in a determined host cell. The vector must therefore contain a promoter, signals of initiation and termination of translation, as well as appropriate regions of regulation of transcription. It must be able to be maintained in a stable manner in the host cell and can optionally have particular signals which specify the secretion of the translated protein. These different elements are chosen and optimized by the person skilled in the art as a function of the host cell used. To this effect, the nucleotide sequences according to the invention can be inserted into autonomous replication vectors in the chosen host, or be integrative vectors of the chosen host.

Such vectors are prepared by methods currently used by the person skilled in the art, and the resulting clones can be introduced into an appropriate host by standard methods, such as lipofection, electroporation, thermal shock, or chemical methods.

The vectors according to the invention are, for example, vectors of plasmidic or viral origin. They are useful for transforming host cells in order to clone or to express the nucleotide sequences according to the invention.

In a preferred embodiment, the vector of the present invention is the plasmid vector pET21 contained in the bacteria which has been deposited according to the Budapest Treaty at the C.N.C.M. (Collection Nationale de Cultures de Microorganismes, Institut Pasteur, Paris, France) the January 12, 2010 under the number I-4272.

This cloned plasmid vector is the vector pET21 wherein the nucleic sequence of the DNA polymerase of the invention has been inserted between the NdeI and XhoI sites of the pET21 plasmid.

The term "expression vector" refers to a recombinant DNA molecule containing the desired coding nucleic acid sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

In another aspect, the present invention relates to a host cell comprising the vector according to the invention.

In another aspect, the present invention is directed to the isolated plasmid pTN2 and the host cell comprising said plasmid pTN2, particularly the recombinant bacteria which has been deposited according to the Budapest Treaty at the C.N.C.M. (Collection Nationale de Cultures de Microorganismes, Institut Pasteur, Paris, France) the February 2, 2010 under the number I-4275.

The DNA polymerase polypeptide of the present invention may be expressed in either prokaryotic or eukaryotic host cells. Nucleic acid encoding the DNA polymerase polypeptide of the present invention may be introduced into bacterial host cells by a number of means including transformation of bacterial cells made competent for transformation by treatment with calcium chloride or by electroporation. If the DNA polymerase polypeptide of the present invention are to be expressed in eukaryotic host cells, nucleic acid encoding the DNA polymerase polypeptide of the present invention may be introduced into eukaryotic host cells by a number of means including calcium phosphate co-precipitation, spheroplast fusion, electroporation and the like. When the eukaryotic host cell is a yeast cell, transformation may be affected by treatment of the host cells with lithium acetate or by electroporation or any other method known in the art. It is contemplated that any host cell will be useful in producing the peptides or proteins or fragments thereof of the invention.

The cells transformed according to the invention can be used in processes for preparation of recombinant polypeptides according to the invention. The processes for preparation of a polypeptide according to the invention in recombinant form, characterized in that they employ a vector and/or a cell transformed by a vector according to the invention, are themselves comprised in the present invention.

Preferably, a cell transformed by a vector according to the invention is cultured under conditions which allow the expression of said polypeptide and said recombinant peptide is recovered.

In another aspect, the present invention relates to a method of producing a DNA polymerase, particularly a recombinant DNA polymerase, said method comprising:
(a) culturing the host cell according to the invention in conditions suitable for the expression of said nucleic acid; and
(b) isolating said DNA polymerase from said host cell.

Preferably, the present invention relates to a method of producing a DNA polymerase, particularly a recombinant DNA polymerase which is soluble in an aqueous solvent, said method comprising:
a) culturing a host cell transformed by a vector according to the invention in conditions suitable for the expression of said nucleic acid;
b) harvesting the cells by centrifugation and resuspending the cells pellet in an aqueous buffer;
c) lysing the cells, preferably by sonication, and, optionally, heating the lysate, preferably at a temperature equal or superior to 65°C, more preferably at a temperature between 70°C and 80°C; and
d) centrifugating and recovering the aqueous soluble fraction containing the recombinant DNA polymerase.

Said host cell can be a prokaryotic or an eukaryotic cell.

As has been said, the host cell can be chosen from prokaryotic or eukaryotic systems. In particular, it is possible to use nucleotide sequences facilitating secretion in such a prokaryotic or eukaryotic system. A vector according to the invention carrying such a sequence can therefore advantageously be used for the production of recombinant proteins, intended to be secreted. In effect, the purification of these recombinant proteins of interest will be facilitated by the fact that they are present in the supernatant of the cell culture rather than in the interior of the host cells.

In another aspect, the present invention encompasses a method of synthesizing a double-stranded DNA molecule comprising:
(a) hybridizing a primer to a first DNA molecule; and
(b) incubating said DNA molecule of step (a) in the presence of one or more deoxyribonucleoside triphosphates or analogs thereof and the polypeptide according to the invention, under conditions sufficient to synthesize a second DNA molecule complementary to all or a portion of said first DNA molecule.

In another aspect, the present invention encompasses a method of synthesizing or amplifying a single-stranded DNA or a double-strand DNA from an unknown DNA template, said method comprising:
optionally, whether it is necessary, beforehand denaturing the double-stranded DNA molecule template; and
   (a) incubating said DNA template in the presence of one or more deoxyribonucleoside triphosphates or analogs thereof and the polypeptide according to the invention or obtained by a method according to the invention having a DNA polymerase and primase activities, under conditions sufficient to synthesize a second DNA molecule complementary to all or a portion of said first DNA molecule.

In another aspect, the present invention encompasses a method of synthesizing a single-stranded DNA molecule comprising:
(a) synthesis of a double-stranded DNA molecule by a method according to the invention; and
(b) denaturing the double-stranded DNA molecule obtained in step (a); and
(c) recovering the single-stranded DNA molecule obtained in step (b).

In another aspect, the present invention encompasses a method for production and/or the amplification of DNA molecules of greater than 5 kilobases (Kb) in length, preferably greater than 10, 15, 20 or 25 Kb, said method implementing a polypeptide of the present invention, particularly the method according to the invention, wherein the first DNA molecule which serve as a template in step (a) is greater than 5, 10, 15 or 20 Kb, preferably greater than 25 Kb.

In the method according to the invention, said deoxyribonucleoside triphosphates are selected from the group consisting of dATP, dCTP, dGTP and dTTP.

In another aspect, the present invention encompasses a method for amplifying a double stranded DNA molecule, comprising:
(a) providing a first and second primer, wherein said first primer is complementary to a sequence at or near the 3'-termini of the first strand of said DNA molecule and said second primer is complementary to a sequence at or near the 3'-termini of the second strand of said DNA molecule;
(b) hybridizing said first primer to said first strand and said second primer to said second strand in the presence of the polypeptide according to the invention, under conditions such that a nucleic acid complementary to said first strand and a nucleic acid complementary to said second strand are synthesized;
(c) denaturing
   - said first and its complementary strands; and
   - said second and its complementary strands; and
(d) repeating steps (a) to (c) one or more times.

It is also preferred that the step of amplifying is performed by PCR, or PCR-like method, or RT-PCR reaction implementing the polypeptide having a DNA polymerase activity (DNA polymerase polypeptide).

"PCR" describes a method of gene amplification which involves sequenced-based hybridization of primers to specific genes within a DNA sample and subsequent amplification involving multiple rounds of annealing (hybridization), elongation and denaturation using a heat-stable DNA polymerase.

"RT-PCR" is an abbreviation for reverse transcriptase-polymerase chain reaction. Subjecting mRNA to the reverse transcriptase enzyme results in the production of cDNA which is complementary to the base sequences of the mRNA. Large amounts of selected cDNA can then be produced by means of the polymerase chain reaction which relies on the action of heat-stable DNA polymerase.

"PCR-like" will be understood to mean all methods using direct or indirect reproductions of nucleic acid sequences, or alternatively in which the labelling systems have been amplified, these techniques are of course known, in general they involve the amplification of DNA by a polymerase; when the original sample is an RNA, it is advisable to carry out a reverse transcription beforehand. There are currently a great number of methods allowing this amplification, for example the so-called NASBA "Nucleic Acid Sequence Based Amplification", TAS "Transcription based Amplification System", LCR "Ligase Chain Reaction", "Endo Run Amplification" (ERA), "Cycling Probe Reaction" (CPR), and SDA "Strand Displacement Amplification", methods well known to persons skilled in the art.

When using mRNA, the method may be carried out by converting the isolated mRNA to cDNA according to standard methods using reverse transcriptase (RT-PCR).

In another aspect, the present invention encompasses a method of preparing cDNA from mRNA, comprising:
(a) contacting mRNA with an oligo(dT) primer or other complementary primer to form a hybrid, and
(b) contacting said hybrid formed in step (a) with the DNA polymerase polypeptide according to the invention and dATP, dCTP, dGTP and dTTP, whereby a cDNA-RNA hybrid is obtained.

The present invention is further directed to a method of preparing dsDNA (double strand DNA) from mRNA, comprising:
(a) contacting mRNA with an oligo (dT) primer or other complementary primer to form a hybrid; and
(b) contacting said hybrid formed in step (a) with the polypeptide according to the invention, dATP, dCTP, dGTP and dTTP, and an oligonucleotide or primer which is complementary to the first strand cDNA;
whereby dsDNA is obtained.

In another aspect, the present invention encompasses a method for determining the nucleotide base sequence of a DNA molecule, comprising the steps of:
(a) contacting said DNA molecule with a primer molecule able to hybridize to said DNA molecule;
(b) incubating said hybrid formed in step (a) in a vessel containing four different deoxynucleoside triphosphates, a DNA polymerase polypeptide according to the invention, and one or more DNA synthesis terminating agents which terminate DNA synthesis at a specific nucleotide base, wherein each said agent terminates DNA synthesis at a different nucleotide base; and
(c) separating the DNA products of the incubating reaction according to size, whereby at least a part of the nucleotide base sequence of said DNA can be determined.

In a preferred embodiment, said terminating agent is a dideoxynucleoside triphosphate.

A DNA synthesis terminating agent which terminates DNA synthesis at a specific nucleotide base refers to compounds, including but not limited to, dideoxynucleosides having a 2',3' dideoxy structure (e.g., ddATP, ddCTP, ddGTP and ddTTP). Any compound capable of specifically terminating a DNA sequencing reaction at a specific base may be employed as a DNA synthesis terminating agent.

In another aspect, the present invention encompasses a method for amplification of a DNA molecule comprising the steps of:
(a) incubating said DNA molecule in the presence of a polypeptide having DNA polymerase according to the invention and a mixture of different deoxynucleoside triphosphates.

In a preferred embodiment, the method for the synthesizing or the amplification of a DNA molecule according to the invention comprises the addition of an enzyme having a helicase activity in order to improve the processivity of the DNA polymerase polypeptide of the present invention.

In a further aspect, the present invention is directed to a kit for sequencing a DNA molecule, comprising:
(a) a first container means comprising the polypeptide according to the invention;
(b) a second container means comprising one or more dideoxyribonucleoside triphosphates; and
(c) a third container means comprising one or more deoxyribonucleoside triphosphates.

The present invention also encompasses a kit for amplifying a DNA molecule, comprising:
(a) a first container means comprising the polypeptide according to the invention; and
(b) a second container means comprising one or more deoxyribonucleoside triphosphates.

The present invention also comprises the use of a polypeptide according to the invention for implementing rolling circle amplification, multiple displacement amplification or protein-primed amplification method.

These particular methods are well known by the skilled person and are for example described in the documents:
Lizardi et al., 1998; Baner et al., 1998; Dean et al., Genome Res., 11, 1095-1099, 2001;
Larsson et al., Nature methods, 1, 227-232, 2004; for isothermal rolling-circle amplification method;
Dean et al., 2002, for multiple displacement amplification method; and
Blanco et al., 1994, for protein-primed amplification method.

In another aspect, the present invention also provides methods for producing anti-DNA polymerase polypeptide of the invention comprising, exposing an animal having immunocompetent cells to an immunogen comprising a polypeptide of the invention or at least an antigenic portion (determinant) of a polypeptide of the invention under conditions such that immunocompetent cells produce antibodies directed specifically against the polypeptide of the invention, or epitopic portion thereof. In one embodiment, the method further comprises the step of harvesting the antibodies. In an alternative embodiment, the method comprises the step of fusing the immunocompetent cells with an immortal cell line under conditions such that a hybridoma is produced.

Such antibodies can be used particularly for purifying the polypeptide of the present invention in a sample where others components are present.

The present invention is finally directed to an apparatus for DNA sequencing or amplification having a reactor comprising a DNA polymerase polypeptide of the present invention.

The following examples and the figures are given for the purpose of illustrating various embodiments of the invention and are not meant to limit the present invention in any fashion.

### Legends of the figures

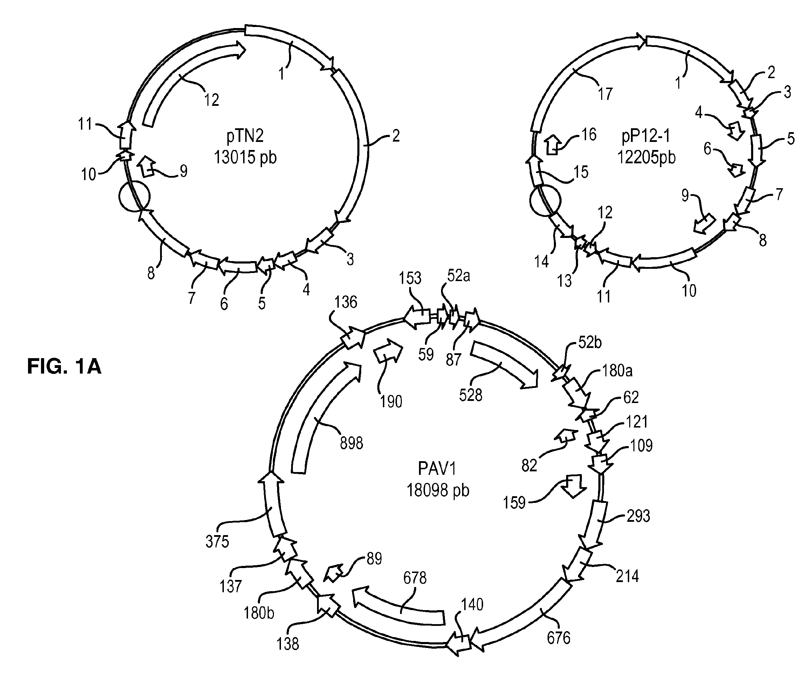

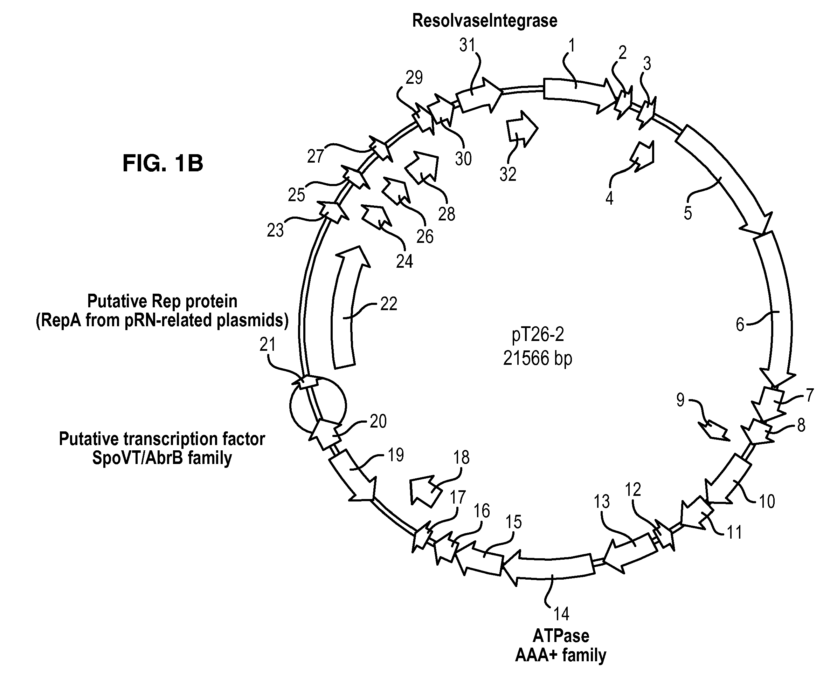

**Figures 1A** **and** **1B****:** Schematic representation of the three new plasmids
   - **Figure 1A****.** pTN2 and pP12-1 were drawn at the same scale together with PAV1 genome. ORFs are numbered and represented as arrows. ORFs encoding homologous proteins have the same color. White ORFs do not have detectable homologues among these three genomes.
   - **Figure 1****B.** pT26-2 with ORFs numbered and represented as arrows. Coloured ORFs encode proteins with expected activity or function.
   - **Figures 1A** **and** **1B****.** Hachured ORFs harbour putative hydrophobic segments. Circles indicate large intergenic regions including putative replication origins.
**Figures 2A and 2B****:** tn2-12p is a DNA polymerase

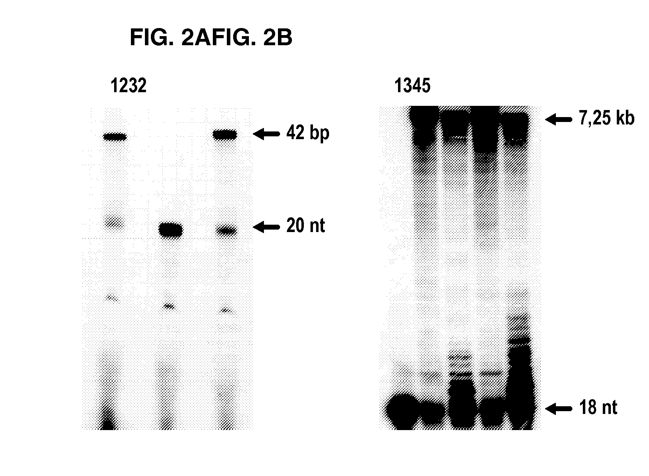

   - **Figure 2A****:** The recombinant protein tn2-12p (15 µM) (lanes 1 and 2) or Taq polymerase (Promega, 0,02U/µl) (lane 3) were incubated with a short primer-template substrate (5'-³²P labeled 20 nt oligonucleotide CGAACCCGTTCTCGGAGCAC (SEQ ID NO: 15) hybridized to 42 nt template TTCTGCACAAAGCGGTTCTGCAGTGCTCCGAGAACGGGTTCG) (SEQ ID NO: 16). Primers are extended in the presence of 0,2 mM dNTPs (lanes 1 and 3) or 0,2 mM NTPs (lane 2) during 20 min at 70°C. In the control reaction with NTPs (line 2) no primer elongation was observed. The buffer used for the polymerization reaction is described in Materials and Methods. Extension products were analyzed on the 16 % denaturating polyacrylamide gel.
   - **Figure 2B****:** The primer extension activity of tn2-12p was assayed at 70°C (lanes 1-3) and 80°C (lanes 4 and 5). A 5'-³²P-labelled 18 nt primer (GTAAAACGACGGCCAGTG) (SEQ ID NO: 17) was hybridized with ssDNA of M13. 10nM of this primer-template substrate were incubated for 20 min either without any proteins (lane 1), either with 10 µM of tn2-12p (lanes 2 and 4) or with 0,05 U/µl of Tag polymerase (lanes 3 and 5) in the presence of 0,2 mM dNTPs at one of the temperature indicated. The buffer used for the polymerization reaction is described in Materials and Methods. Extension products were analyzed on the 16 % denaturating polyacrylamide gel.

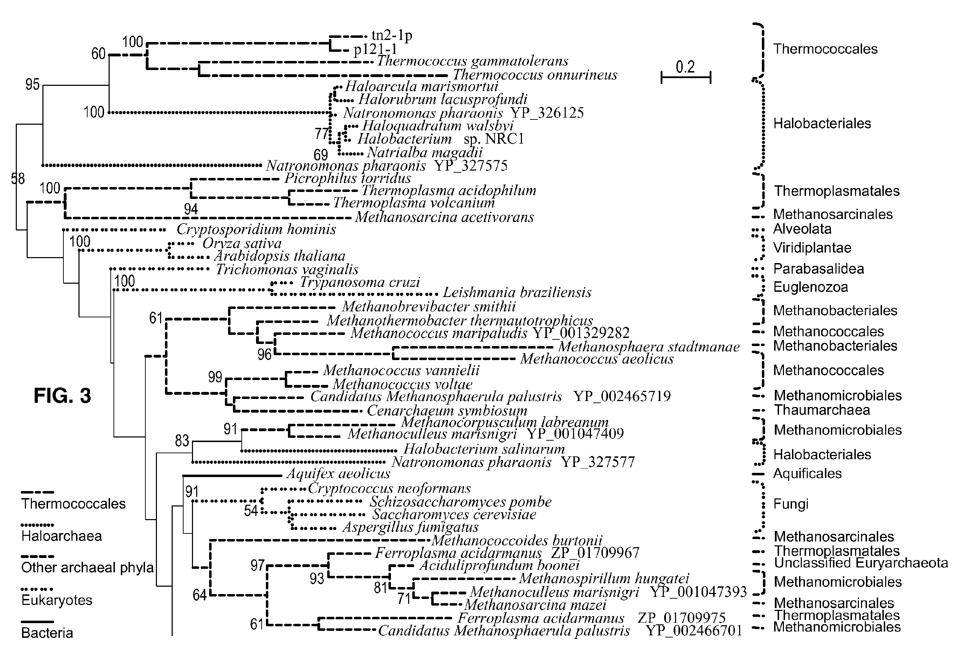

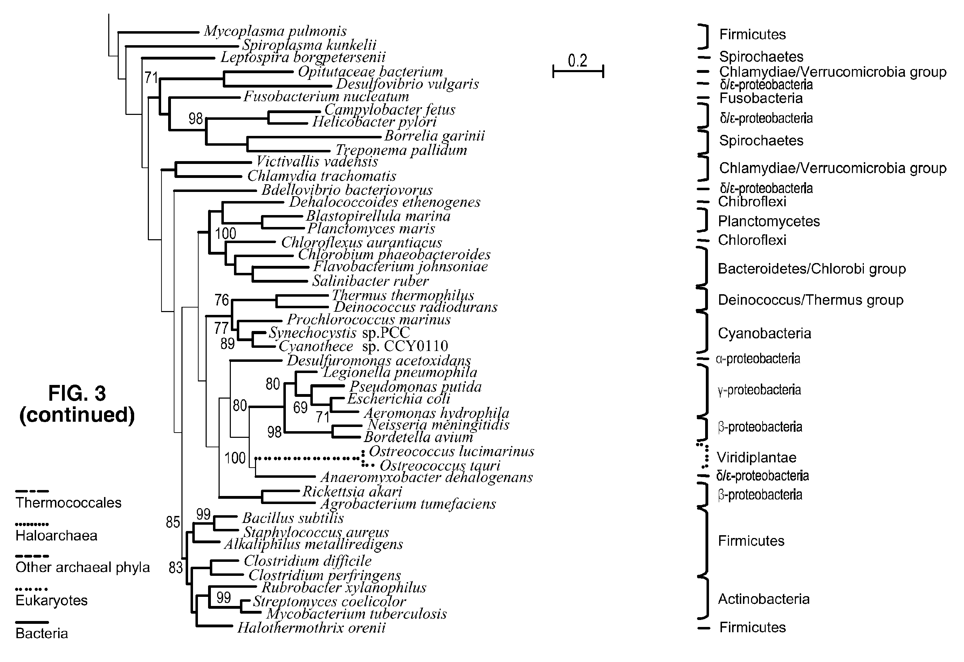

**Figure 3****:** Phylogenic trees of SFI Helicase.

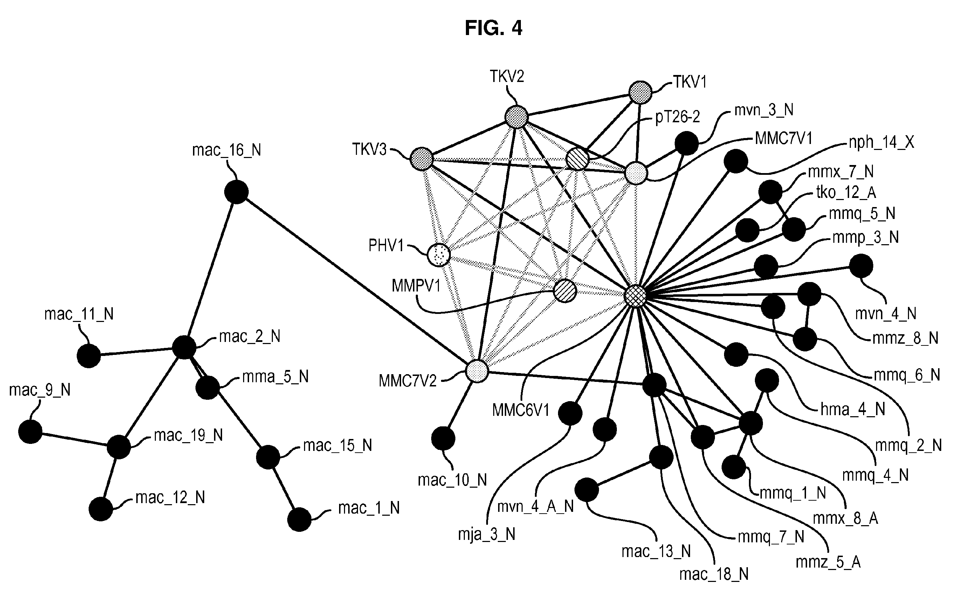

**Figure 4****:** CAGs families.
   The plasmid pT26-2 (yellow) and related elements of the family, TKV2 (blue), TKV3 (blue), PHV1 (light blue), MMPV1, (orange) MMC7V1 (pink), MMC7V2 (pink) and MMC6V1 (mauve) share a core of six gene and are linked with bold lines. They share four genes with a string of CAGs present in genomes of Thermococcales, Methanococcales and Methanosarcines ( ) and their edges are colored in dark gray. These elements may show one or several links with integrated elements detected in other archaea (Cortez et al. Genome Biology 2009). mmp is *M. marpaludis,* mmq is *M. maripaludis* C5, mmx is *M. maripaludis* C6, mmz is *M. maripaludis* C7, mja is *M. jannaschii* and mvn is *M. vannielii.* Methanosarcinales: mac is *M. acetivorans.*

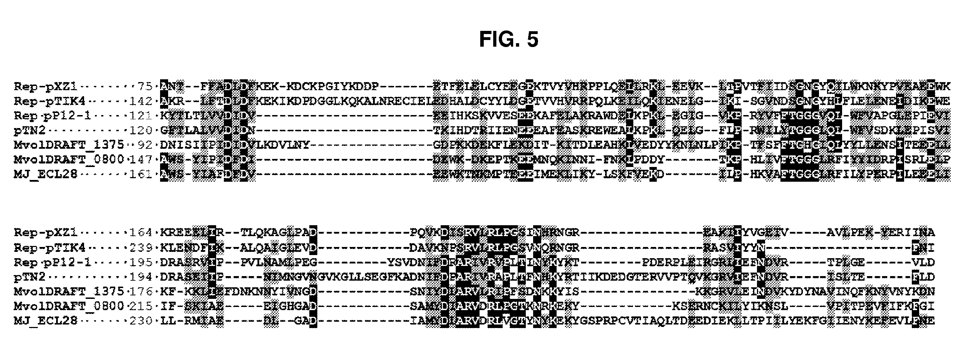

**Figure 5****:** Alignment DNA polymerases.

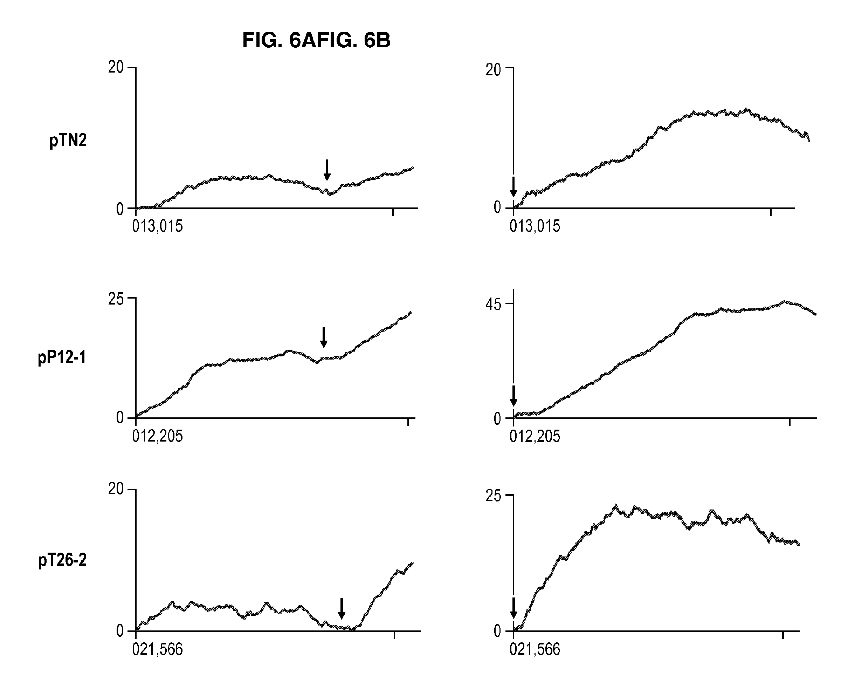

**Figures 6A and 6B****:** Cumulative GC (Figure 6A) and AT (Figure 6B) skews graphics for pTN2, pP12-1 and pT26-2 plasmid genomes.

### EXAMPLE 1: Materials and Methods

### Stains cultivation

*Thermococcus nautilus* sp. 30-1, *Pyrococcus sp.* 12-1, and *Thermococcus sp.* 26-2, were isolated from single colonies by plating on Gelrite an enrichment culture obtained from fragments of chimneys collected at deep sea hydrothermal vents (- 2.630 m) in the East Pacific ocean (Lepage et al., 2004). Cells were grown in Zillig's broth (ZB) made anaerobic by addition of Na2S (0.1 mg/liter), with sulfur flowers S (Fisher Scientific) as previously described (Lepage et al., 2004). The cultures were incubated in Penny's flasks at 85°C for *Thermococcus sp.* 26-2 and *T. nautilus,* or 95°C for *Pyrococcus sp.* 12-1.

### Plasmid isolation

Plasmids were obtained by alkaline extraction procedure as described previously (Soler et al, 2007) from 50 mL culture of *T. nautilus* 30-1, *Pyrococcus* 12-1 or *Thermococcus* 26-2 cells that had been grown until stationary phase. Plasmids were sequenced by Fidelity Systems, USA. The complete sequence of have been sequenced and annotated. Concerning the pTN2 of *T. nautilus* 30-1, see SEQ ID NO: 1 and the Table 1.

### Sequence analysis

BLAST and PSI-BLAST searches were performed in the NCBI non redundant (nr) databank using the following web sites: http://www.ncbi.nlm.nih.gov/BLAST/ and http://www-archbac.u-psud.fr/genomics/GenomicsToolbox.html/. Hydrophobic regions were detected using TMpred and TMHMM prediction programs located at the web site http://www.expasy.org/tools/. ORFs were identified with a minimum of 50 amino acids length and with one of the for initiator codons (ATG, GTG, CTG, TTG). The exact position of each initiator codon was then checked individually (and manually modified if required) depending on the position of putative Shine-Dalgarno sequence located upstream.

### pTN2 sequence annotation.

**See the Table 1.**

**Table 1. Annotation of pTN2 plasmid from the strain Thermococcus nautilus 30/1.**

| **ORF** | **Putative protein** | **Size** **(kDa)** | **ORF position** **(start-stop)** | **RBS and start codon** | **Homologue in another plasmid** | **% identity, length of BLAST alignment (aa)** | **Putative motives and function** |
|---|---|---|---|---|---|---|---|
| 1 | tn2-1p | 66,1 | 1-1710 (SEQ ID NO: 2) | aggagggggtggtcggGTG (SEQ ID NO: 18) | p12-1p | 69%, 569 | UvrD/Rep/PcrA superfamily I type helicase |
| 2 | tn2-2p | 104,9 | 1785-4622 (SEQ ID NO: 3) | ggggggatttgtATG (SEQ ID NO: 19) | - | - | Putative coiled-coil domains |
| 3 | tn2-3p | 20,3 | 4841-5365 (SEQ ID NO: 4) | tgggggatgacgATG (SEQ ID NO: 20) | p12-14p | 46%, 173 | |
| 4 | tn2-4p | 15,2 | 5590-5979 (SEQ ID NO: 5) | gggtggtgtcgtaattATG (SEQ ID NO: 21) | - | - | Putative helix-turn-helix domain |
| 5 | tn2-5p | 11,2 | 5983-6279 (SEQ ID NO: 6) | aggggaggtgtaaccgATG (SEQ ID NO: 22) | - | - | |
| 6 | tn2-6p | 26,4 | 6285-6959 (SEQ ID NO: 7) | agaggtgtaaaacaaATG (SEQ ID NO: 23) | - | - | |
| 7 | tn2-7p | 19,5 | 6959-7477 (SEQ ID NO: 8) | aggaggggttatgATG (SEQ ID NO: 24) | p12-9p | 41%, 41 | |
| 8 | tn2-8p | 43 | 7542-8648 (SEQ ID NO: 9) | tggaggtgccgagcGTG (SEQ ID NO: 25) | p12-10p | 46%, 367 | ATP binding site, that is related to ABC transporters |
| 9 | tn2-9p | 16 | 9184-9597 (SEQ ID NO: 10) | agggggtactcataATG (SEQ ID NO: 26) | - | - | |
| 10 | tn2-10p | 6,7 | 9551-9730 (SEQ ID NO: 11) | cggaggaggataATG (SEQ ID NO: 27) | - | - | |
| 11 | tn2-11p | 18,4 | 9763-10251 (SEQ ID NO: 12) | tggaggtgatgtaaATG (SEQ ID NO: 28) | - | - | |
| 12 | tn2-12p (SEQ ID NO: 14) | 106,8 | 10248-4 (SEQ ID NO: 13) | agggggtgaaagtATG (SEQ ID NO: 29) | p121-17 | 55%, 922 | DNA polymerase/primase activities |

### Phylogenetic analyses

Homologous sequences were recovered by BLAST searches, and multiple alignments were performed with the selected sequences using MUSCLE program (Edgar, 2004). Only homologous positions were used to build unrooted maximum likelihood trees using PHYML (Guindon and Gascuel, 2003). The JTT model of amino acid substitution was chosen, and a gamma correction with 4 discrete classes of sites was used. The alpha parameter and the proportion of invariable sites were estimated. The robustness of trees was tested by non parametric bootstrap analysis using PHYML.

### In silico identification of integrated mobile element and CAGs families

Mobile elements integrated in cellular genome were identified as Cluster of Atypical Genes (CAGs) according to Cortez *et al.,* (2009) (Cortez *et al.,* 2009). Briefly, archaeal genomes were analyzed with a species-specific Markov model in order to obtain the list of atypical genes. We then searched for atypical genes that cluster together, since these may be recently integrated foreign elements. We used a sliding window of ten ORFs that moved along the genome sequence. A CAG was defined when 7 or more ORFs in that window showed an atypical composition. To define CAGs families, Blast searches were performed with all the ORFs contained in our CAGs with an e-value of 10e⁻²⁰. We then generated several topological networks of CAGs by drawing a line between pairs of CAGs that share a defined number of ORFs (from two up to 6). The graphical representation was obtained with the Cytoscape program (http://www.cytoscape.org).

### DNA polymerase assay

Two different 5'-labelled primer-template systems were used (see Figures 2A and 2B legends). The standard polymerase assay contained 10 nM primer-template substrate, 10-15 5µM of recombinant protein tn2-12p in 10 mM Tris-HCl pH 9, 1 mM DTT, 50 mM KCI, 0,1% Triton x100™, 50 mM MgCl₂ and 0,2 mM dNTPs. The protein was diluted in 50 mM Tris-HCl pH 8, 1 mM DTT, 100 mM NaCl, 0,1 mM EDTA. The reactions were allowed to proceed for 20 min at 70 or 80°C and were analyzed by denaturating PAGE.

### EXAMPLE 2: Isolation of three new plasmids from Thermococcales

Plasmids pTN2, pP12-1 and pT26-2 were isolated from three strains of Thermococcales that were purified from samples collected at three different deep-sea hot vents located in the East-Pacific ridge during the AMISTAD cruise (Lepage et al., 2004). The strain 30-1, that harbours the plasmid pTN2, has been tentatively described as the type strain of a new species, *Thermococcus nautilus* (Soler et al., 2007). The strain 26-2, that harbours the plasmid pT26-2 is closely related to *T. nautilus* sp. 30-1 by 16S rRNA analysis and could belong to the same species, although it exhibits a very different RAPD profile (Lepage et al., 2004). Finally, the strain 12-1, which harbours the plasmid pP-12-1 belongs to a RAPD group that includes a *Pyrococcus* species (isolate 32-4) and will be thereafter called *Pyrococcus* sp. 12-1. The three plasmids are circular and their sizes are 13015, 12205, and 21566 bp, respectively. The plasmids sequences have a GC content of around 47.5, 44.6 and 49.6 % for pTN2, pP12-1 and pT26-2, respectively, which are close to GC% of Thermococcales chromosomal DNA (40-50%). By in silico analysis, we identified 12, 17 and 32 putative ORFs in pTN2, pP12-1 and p26-2, respectively Although pTN2 was isolated from a Thermococcus species and pP12-1 from a Pyrococcus species, they turned out to be evolutionary related, since they share six homologous genes. In contrast, although plasmids pTN2 and pT26-2 are present in two closely related strains (30-1 and 26-2), they turned out to be completely unrelated to each other.

All ORFs of pTN2 and pP12-1 are located on the same DNA strand with only two exceptions detected in pP12-1. Similarly, 30 of the 32 pT26-2 ORFs are transcribed in the same direction (Figure 1B). We though to predict the position of the replication origin of these three plasmids by performing cumulative GC skew analysis (Grigoriev, 1998). This method is based on the general observation that GC content usually differ between the leading and lagging strands of replication forks (Lobry, 1996). The cumulative GC skews graphics for the three plasmids show GC frequency inversion producing V-like curves. Strikingly, the minima are located in the larger intergenic regions for each of the three plasmids (circles Figures 1A and 1B). These intergenic regions are among the most AT-rich of the three plasmids and contain many direct and inverted repeats; features that are general characteristics of plasmid replication origins.

### EXAMPLE 3: The pTN2 plasmid family

We identified 12 and 17 putative ORFs in the plasmids pTN2 and pP12-1 sequences, respectively (see material and methods). All ORFs of pTN2 and most ORFs of pP12-1 are located on the same strand with only two exceptions in the case of pP12-1. The plasmids pTN2 and pP12-1 share six homologous ORFs, including two contiguous large ORFs encoding proteins of around 66 and 105 kDa, respectively (Figures 1A and 1B). The first one (tn2-1p and p12-1p) corresponds to a helicase of the superfamily I (SFI). These helicases include the well characterized bacterial helicases UvrD, PcrA and Rep. They are widespread in the three domains with many representatives in several bacterial and archaeal phyla, and in a few eukaryotic ones. The closest relative of tn2-1p and p12-1p is encoded in the genome of Thermococcus onnurineus. Other close homologues are encoded in the genomes of euryarchaea from various orders (Halobacteriales, Thermoplasmatales, Methanosarcinales, Methanomicrobiales, Methanobacteriales, Methanococcales) and one in the genomes of a Thaumarchaeon (Cenarchaeum symbiosum) (for definition of Thaumarchaea, see Brochier-Armanet et al., 2008). We have built a phylogenetic tree of this helicase family with a selection of sequences from the three domains (at least one sequence per represented phylum) (Figure 4). In this tree, the closest relatives of the pTN2 and pP12-1 helicases are encoded in the genomes of *Thermococcus gammatolerans* and *Thermococcus onnurineus.* The four helicases from Thermococcales form a monophyletic group with seven helicases from Haloarchaea. Overall, the archaeal SFI helicases tree is not congruent with the archaeal specie tree based on ribosomal proteins or RNA polymerase subunits (Brochier-Armanet *et al.,* 2008; Brochier *et al.,* 2005). The archaeal sequences form five monophyletic groups and Archaea of the same orders are often present in different groups (Figure 4). The SFI helicase of the thaumarchaeon *Cenarchaeum symbiosum* is grouped with some Methanococcales (phylum Euryarchaea). Furthermore, several groups of eukaryotic helicases (from plants and protists) are interspersed between archaeal groups, whereas helicases from fungi form a monophyletic group with sequences from Methanomicrobiales and Thermoplasmatales. This phylogeny is difficult to interpret because lengths are highly variable, probably inducing phylogenetic artefacts. Interestingly, the "cellular" homologue of pTN2-type helicases in *T. gammatolerans* is located in an integrated virus-like element, TGV2 (Zivanovic *et al.,* 2009). In *Methanococcus maripaludis* strain C6 (MMC6V1) and *Methanococcus maripaludis* strain C7 (MMC7V2) the genes encoding pTN2-type helicases are located in predicted integrated elements of plasmid/virus origin that have been identified *in silico,* based on their dinucleotide sequence composition (Cortez *et al.,* 2009). These observations suggest that some archaeal, and possibly eukaryotic SFI helicases, originated from viruses and plasmids and were transferred independently into various lineages, explaining the incongruence between their phylogeny and the classical archaeal and eukaryotic phylogenies. The frequent grouping of Archaea from different phyla in the same clade furthermore suggests that horizontal gene transfers of genes encoding these helicases also sometimes occurred in Archaea. The bacterial helicases of the SFI helicase family are quite well separated from Archaeal and Eukaryotic, with the exception of those from the bacterium *Aquifex aeolicus* that branches with the group mixing some helicases from Archaea and Fungi. Bacterial UvrD-like helicases form a monophyletic group (with two representatives in the eukaryotes *Ostreococcus,* a probable case of gene transfer from a mitochondrial or plastid genome). However, as in the case of Archaea, Bacteria from the same phylum are often dispersed in different parts of the tree, suggesting that the genes encoding these helicases have been also sometimes transferred between bacterial phyla.

The second large ORFs conserved between pTN2 and pP12-1 (tn2-12p and p12-17p, respectively) encodes proteins of 107 and 103 kDa, respectively. Psi-BLAST searches using tn2-12p as query gave no significant result). Interestingly, we noticed among hits of the first PSI-BLAST iterations the putative Rep protein of the recently described plasmid pXZ1 from *Sulfolobus islandicus* (Peng, 2008). BLAST search with the sequence of the pXZ1 Rep protein then retrieved the Rep protein of the plasmid pTIK4 from *Sulfolobus neozelandicus* (Greve *et al.,* 2005). We could align manually the N-terminal regions of tn2-12p and p12-17p with those of the Rep proteins of pXZ1 and pTIK4 (Figure 5). We then noticed that these proteins exhibit a conserved DhD motif, known to be present in the active sites of many DNA polymerases, primases and/or nucleotidyl transferases (Iyer *et al.,* 2005).

### EXAMPLE 4: Cloning, expression, and purification of tn2-12p

The tn2-12p protein has been expressed in *E. coli,* particularly to test and to demonstrate the DNA polymerase, primase and transferase activities exhibited by the protein *in vitro.*

The coding sequence of tn2-12p was amplified by PCR from plasmidic DNA isolated from the strain *Thermococcus nautilus* 30/1, and the amplified fragment was cloned in a pET30a plasmid allowing fusion of a 6His tag at 3' end. Expression was done at 37°C using the *E. coli* Rosetta (DE3) pLysS strain and the 2xYT medium (BIO 101 Inc.). When the cell culture reached an OD_{600 nm} of 0.8, induction at 15°C was performed overnight with 0.5 mM IPTG (Sigma). Cells were harvested by centrifugation and resuspended in buffer A (20 mM Tris-HCl pH 7.5, 200 mM NaCl, 5 mM beta-mercaptoethanol). Cell lysis was completed by sonication and the lysate was heated for 20 min at 70°C before centrifugation at 20,000 rpm for 20 min. The soluble fraction was loaded on a NiNTA column (Qiagen Inc.) equilibrated with buffer A. The protein was eluted with imidazole and subsequently loaded on a heparin column (GE Healthcare) equilibrated against buffer A' (20 mM tris Tris-HCl pH 7.5, 50 mM NaCl, 5 mM beta-mercaptoethanol). Elution was performed using a gradient between buffer A' and buffer B (20 mM tris Tris-HCl pH 7.5, 1M NaCl, 5 mM beta-mercaptoethanol). The tn2-12p protein was eluted at about 0.9 M NaCl. Eluted fractions were pooled and loaded on a Superdex200 column (Amersham Pharmacia Biotech) equilibrated against buffer A supplemented with 10 mM beta-mercaptoethanol. The homogeneity of the protein sample was checked by SDS-PAGE.

### EXAMPLE 5: Demonstration of the thermostable DNA polymerase, primase and transferase activities of the tn2-12p recombinant protein

The purified recombinant tn2-12p protein was first incubated with dNTPs and 5'-labelled 20 nt DNA primer hybridized to a complementary 42 nucleotides DNA template. As expected, if our prediction was correct, the primer was efficiently extended up to the full length of the template (Figure 2A). The DNA polymerizing activity of tn2-12p requires the presence of dNTPs and a DNA template (data not shown) and it is ATP independent. No extension was observed when the dNTPs were replaced by NTPs. The optimum temperature of the DNA polymerase activity was assayed on M13 DNA primed with a 18 nucleotides DNA primer (Figure 2B). At 65°C the activity of tn2-12p was lower (data not shown) than at 70°C and 80°C, which therefore excludes the possibility that the observed activity could be due to the presence of some E. coli proteins co-purified with tn2-12p. The protein tn2-12p is able to extend the primer up to several kilobases at 20 min of the reaction times and the obtained DNA shows the same pattern as that synthesized by the Taq polymerase. This result indicates that tn2-12p can produce long extension products and its activity is independent of the presence of additional proteins. Additionally to its DNA polymerase activity, the tn2-12p protein exhibits primase and nucleotidyl-transferase activities. Preliminary sequence analyses based on hand-made alignments indicate that homologues of these DNA polymerases are widespread in Archaea and Bacteria (always encoded by plasmids, viruses or integrated elements). In particular, we found one copy in T. gammatolerans (TGAM_1629) and in T. onnurineus (TON_1379).

The DNA polymerase/primase activities found associated to tn2-12p, are consistent with the idea that tn2-12p and the homologous protein p12-17p correspond to the Rep proteins of the plasmids pTN2 and pP12-1, respectively. In the case of pRN1, the DNA polymerase domain of RepA is located in the N-terminal part of the protein and is fused to a helicase domain (SFIII) in C-terminal. In the case of the pTN2 and pP12-1 Rep proteins, Psi-BLAST analysis suggests that the DNA polymerase domain is also located in their N-terminal part (which contains the two conserved aspartate residues probably present in the active site). They also indicate that, as in the case of pRN1-type RepA, these polymerase domains are fused to large C-terminal domains. However, Psi-BLAST analysis using these domains as queries retrieved no hit in databases.

Using the Multalin program (Corpet, 1988), we found that p12-17 and the pXZ1 Rep protein also shares a short stretch of highly conserved amino-acids located in the middle of these proteins. PSI-BLAST searches using these central conserved regions of pTN2 and pXZ 1 Rep proteins as queries recovered proteins with the S1 RNA binding motif, suggesting that these regions could bind nucleic acid. It is tempting to speculate that the Rep proteins of pTN2 and pP12-1 are formed by the fusion of a novel type of polymerase/primase in N-terminal and an origin of replication binding domain (corresponding to the S1 nucleic acid motive). As previously mentioned, the genes encoding the SF1 helicase and the Rep protein are contiguous in both pTN2 and pP12-1. Interestingly, combining the DNA polymerase/primase and the putative origin binding activity of these Rep proteins with the activity of the neighbouring helicase would reconstitute a fully operational system for plasmid DNA replication by the theta mode.

### EXAMPLE 6: Prediction of the position of the replication origin of pTN2 and pP12-1

To predict the position of the replication origin of pTN2 and pP12-1, we performed cumulative GC and AT skews analyses, applying the following formulas: (G-C)/(G+C) and (A-T)/(A+T) (Grigoriev, NAR, 1998). This method is based on the general observation that GC and AT content usually differ between the leading and lagging strands of replication forks. Cumulative GC and AT skews graphics for pTN2 and pP12-1 genomes show that the two plasmids have a GC frequency inversion exactly were a large intergenic region is found (see Figure 6A). These results indicate that these large intergenic regions likely harbour the origin of replication.

### Conclusion

All plasmids previously isolated from Thermococales were small rolling circle plasmids (Erauso *et al.,* 1996; Marsin & Forterre, 1998; Marsin & Forterre, 1999; Soler *et al.,* 2008). Here, we report the isolation, sequencing and characterization of three new plasmids from Thermococcales that are larger and probably replicate via the theta mode. These plasmids do not encode homologue of Rep endonucleases known to initiate rolling-circle replication. The plasmids pTN1 and pP12-1 encode a DNA polymerase fused to a domain of unknown function, forming a new type of plasmidic Rep proteins. These proteins are homologous to the RepA protein of the plasmids **pTIK4** from *S. neozelandicus* and pXZ1 from a *S. solfataricus* species (Peng, 2008) and to proteins encoded by integrated elements present in the genome of Methanococcales. They form a new family of archaeal RepA proteins common to mobile elements present in both Euryarchaea and Crenarchaea. The DNA polymerase domain of these RepA proteins have no detectable sequence similarities with those of previously known DNA polymerases, including the DNA polymerase discovered by Georg Lipps in pRN1 (Lipps et al., 2003), indicating that these proteins could be the prototypes of a new DNA polymerase family. As in the case of the pRN1 polymerases, the DNA polymerase of pTN2 exhibits a primase activity and could be used for the initiation as well as for the elongation step of plasmid DNA replication. The genes encoding the Rep-DNA polymerase in pTN2 and pP12-1 are contiguous to the genes encoding the SFI helicases. It is tempting to speculate that these large Rep proteins also bears an origin binding activity. The combination of the DNA polymerase/primase activity of these Rep proteins, together with the activity of the helicase SFI, and an origin recognition activity would reconstitute a fully operational system for plasmid DNA replication by the theta mode.

The plasmids pTN2 and pP12-1 can be considered as the prototypes of a new plasmid family, presently specific for Thermococcales (thereafter dubbed the pTN2 family). The gene encoding the new type of DNA primase/polymerase of these two plasmids is located directly downstream a gene encoding a helicase of the SFI family. In addition, both plasmids encode a probable 3' to 5' exonuclease domain of the DnaQ family (that could be involved in proof-reading), and a regulator of the CopG family. These plasmids could become interesting models to study plasmid replication and expression both in vitro and in vivo in hyperthermophilic archaea, much like the pRN1 plasmid from *Sulfolobus islandicus* has been successfully use to study plasmid replication in hyperthermophilic crenarchaea (Lipps, 2009).

### References

Albers SV, Jonuscheit M, Dinkelaker S, Urich T, Kletzin A, Tampe R, Driessen AJ & Schleper C. (2006). Production of recombinant and tagged proteins in the hyperthermophilic archaeon Sulfolobus solfataricus, App1 Environ Microbiol, 72, 102-11.
Arnold HP, She Q, Phan H, Stedman K, Prangishvili D, Holz I, Kristjansson JK, Garrett R & Zillig W. (1999). The genetic element pSSVx of the extremely thermophilic crenarchaeon Sulfolobus is a hybrid between a plasmid and a virus, Mol Microbiol, 34, 217-26.
Baliga NS, Bonneau R, Facciotti MT, Pan M, Glusman G, Deutsch EW, Shannon P, Chiu Y, Weng RS, Gan RR, Hung P, Date SV, Marcotte E, Hood L & Ng WV. (2004). Genome sequence of Haloarcula marismortui: a halophilic archaeon from the Dead Sea, Genome Res, 14, 2221-34.
Basta T, Smyth J, Forterre P, Prangishvili D & Peng X. (2009). Novel archaeal plasmid pAH1 and its interactions with the lipothrixvirus AFV1, Mol Microbiol, 71, 23-34.
Berkner S, Grogan D, Albers SV & Lipps G. (2007). Small multicopy, non-integrative shuttle vectors based on the plasmid pRN1 for Sulfolobus acidocaldarius and Sulfolobus solfataricus, model organisms of the (cren-)archaea, Nucleic Acids Res, 35, e88.
Cortez et al. Genome Biol. 2009 Jun 16; 10(6):R65.
del Solar G, Giraldo R, Ruiz-Echevarria MJ, Espinosa M & Diaz-Orejas R. (1998). Replication and control of circular bacterial plasmids, Microbiol Mol Biol Rev, 62, 434-64.
Edgar RC. (2004). MUSCLE: a multiple sequence alignment method with reduced time and space complexity, BMC Bioinformatics, 5, 113.
Erauso G, Marsin S, Benbouzid-Rollet N, Baucher MF, Barbeyron T, Zivanovic Y, Prieur D & Forterre P. (1996). Sequence of plasmid pGT5 from the archaeon Pyrococcus abyssi: evidence for rolling-circle replication in a hyperthermophile, J Bacteriol, 178, 3232-7.
Erauso G, Stedman KM, van de Werken HJ, Zillig W & van der Oost J. (2006). Two novel conjugative plasmids from a single strain of Sulfolobus, Microbiology, 152, 1951-68.
Greve B, Jensen S, Brugger K, Zillig W & Garrett RA. (2004). Genomic comparison of archaeal conjugative plasmids from Sulfolobus, Archaea, 1, 231-9.
Greve B, Jensen S, Phan H, Brugger K, Zillig W, She Q & Garrett RA. (2005). Novel RepA-MCM proteins encoded in plasmids pTAU4, pORA1 and pTIK4 from Sulfolobus neozealandicus, Archaea, 1, 319-25.
Harriott OT, Huber R, Stetter KO, Betts PW & Noll KM. (1994). A cryptic miniplasmid from the hyperthermophilic bacterium Thermotoga sp. strain RQ7, J Bacteriol, 176, 2759-62.
Krupovic M & Bamford DH. (2008). Archaeal proviruses TKV4 and MVV extend the PRD1-adenovirus lineage to the phylum Euryarchaeota, Virology, 375, 292-300.
Lepage E, Marguet E, Geslin C, Matte-Tailliez O, Zillig W, Forterre P & Tailliez P. (2004). Molecular diversity of new Thermococcales isolates from a single area of hydrothermal deep-sea vents as revealed by randomly amplified polymorphic DNA fingerprinting and 16S rRNA gene sequence analysis, Appl Environ Microbiol, 70, 1277-86.
Lipps G. (2004). The replication protein of the Sulfolobus islandicus plasmid pRN1, Biochem Soc Trans, 32, 240-4.
Lipps G. (2006). Plasmids and viruses of the thermoacidophilic crenarchaeote Sulfolobus, Extremophiles, 10, 17-28.
Lipps G, Ibanez P, Stroessenreuther T, Hekimian K & Krauss G. (2001a). The protein ORF80 from the acidophilic and thermophilic archaeon Sulfolobus islandicus binds highly site-specifically to double-stranded DNA and represents a novel type of basic leucine zipper protein, Nucleic Acids Res, 29, 4973-82.
Lipps G, Rother S, Hart C & Krauss G. (2003). A novel type of replicative enzyme harbouring ATPase, primase and DNA polymerase activity, Embo J, 22, 2516-25.
Lipps G, Stegert M & Krauss G. (2001b). Thermostable and site-specific DNA binding of the gene product ORF56 from the Sulfolobus islandicus plasmid pRN1, a putative archael plasmid copy control protein, Nucleic Acids Res, 29, 904-13.
Lipps G, Weinzierl AO, von Scheven G, Buchen C & Cramer P. (2004). Structure of a bifunctional DNA primase-polymerase, Nat Struct Mol Biol, 11, 157-62.
Lipps G. Molecular biology of the pRN1 plasmid from Sulfolobus islandicus. Biochem Soc Trans. 2009 Feb;37(Pt 1):42-5.
Lucas S, Toffin L, Zivanovic Y, Charlier D, Moussard H, Forterre P, Prieur D & Erauso G. (2002). Construction of a shuttle vector for, and spheroplast transformation of, the hyperthermophilic archaeon Pyrococcus abyssi, Appl Environ Microbiol, 68, 5528-36.
Marsin S & Forterre P. (1998). A rolling circle replication initiator protein with a nucleotidyl-transferase activity encoded by the plasmid pGT5 from the hyperthermophilic archaeon Pyrococcus abyssi, Mol Microbiol, 27, 1183-92.
Marsin S & Forterre P. (2001). pGT5 replication initiator protein Rep75 from Pyrococcus abyssi, Methods Enzymol, 334, 193-204.
Paull TT. Saving the ends for last: the role of pol mu in DNA end joining. Mol Cell., 2005; 19(3):294-6.
Peng X. (2008). Evidence for the horizontal transfer of an integrase gene from a fusellovirus to a pRN-like plasmid within a single strain of Sulfolobus and the implications for plasmid survival, Microbiology, 154, 383-91.
Peng X, Holz I, Zillig W, Garrett RA & She Q. (2000). Evolution of the family of pRN plasmids and their integrase-mediated insertion into the chromosome of the crenarchaeon Sulfolobus solfataricus, J Mol Biol, 303, 449-54.
Santangelo TJ, Cubonova L & Reeve JN. (2008). Shuttle vector expression in Thermococcus kodakaraensis: contributions of cis elements to protein synthesis in a hyperthermophilic archaeon, Appl Environ Microbiol, 74, 3099-104.
Sato T, Fukui T, Atomi H & Imanaka T. (2005). Improved and versatile transformation system allowing multiple genetic manipulations of the hyperthermophilic archaeon Thermococcus kodakaraensis, Appl Environ Microbiol, 71, 3889-99.
She Q, Singh RK, Confalonieri F, Zivanovic Y, Allard G, Awayez MJ, Chan-Weiher CC, Clausen IG, Curtis BA, De Moors A, Erauso G, Fletcher C, Gordon PM, Heikampde Jong I, Jeffries AC, Kozera CJ, Medina N, Peng X, Thi-Ngoc HP, Redder P, Schenk ME, Theriault C, Tolstrup N, Charlebois RL, Doolittle WF, Duguet M, Gaasterland T, Garrett RA, Ragan MA, Sensen CW & Van der Oost J. (2001). The complete genome of the crenarchaeon Sulfolobus solfataricus P2, Proc Natl Acad Sci U S A, 98, 7835-40.
Soler N, Justome A, Quevillon-Cheruel S, Lorieux F, Le Cam E, Marguet E & Forterre P. (2007). The rolling-circle plasmid pTN1 from the hyperthermophilic archaeon Thermococcus nautilus, Mol Microbiol, 66, 357-70.
Soppa J. (2006). From genomes to function: haloarchaea as model organisms, Microbiology, 152, 585-90.
Stedman KM, She Q, Phan H, Arnold HP, Holz I, Garrett RA & Zillig W. (2003). Relationships between fuselloviruses infecting the extremely thermophilic archaeon Sulfolobus: SSV1 and SSV2, Res Microbiol, 154, 295-302.
Tatusova TA, Madden TL. BLAST 2 Sequences, a new tool for comparing protein and nucleotide sequences. FEMS Microbiol Lett. 1999 May 15; 174(2):247-50.
Ward DE, Revet IM, Nandakumar R, Tuttle JH, de Vos WM, van der Oost J & DiRuggiero J. (2002). Characterization of plasmid pRT1 from Pyrococcus sp. strain JT1, J Bacteriol, 184, 2561-6.

## Claims

1. An isolated polypeptide, wherein:
a) the sequence of said polypeptide comprises the three following motifs:
DhD;
S/TG-GhQ/H; and
D---D--RhhRhP---N, and
b) said polypeptide harbours DNA polymerase and primase activities.

2. A polypeptide according to claim 1, wherein said polypeptide has been isolated from a hyperthermophilic Archaea, preferably from *Thermococcus sp,* or from hyperthermophilic Bacteria, said polypeptide exhibiting a DNA polymerase activity at a temperature comprised between 60°C and 90°C.

3. A polypeptide according to claim 1 or 2, wherein said polypeptide is encoded by a nucleic acid selected from the group consisting of:
- a nucleic acid fragment of the *Thermococcus nautilus* pTN2 plasmid, said pTN2 plasmid having the sequence SEQ ID NO: 1, or of a variant thereof having at least 70 % identity with the sequence SEQ ID NO: 1; and
- a nucleic acid fragment of the *Thermococcus nautilus* pTN2 plasmid ORF12 having the sequence SEQ ID NO: 13, or a variant thereof having at least 70 % identity with the sequence SEQ ID NO: 13.

4. A polypeptide according to one of claims 1 to 3, wherein said polypeptide further exhibits a reverse transcriptase activity.

5. A polypeptide according to one of claims 1 to 4, wherein said polypeptide comprises a polypeptide selected from the group of polypeptides consisting of:
a) the polypeptide having the amino acid sequence SEQ ID NO: 14;
b) a fragment of a) having a DNA polymerase activity or/and a DNA primase activity and/or a nucleotidyl transferase activity;
c) a polypeptide having sequence which is at least 70 % identity after optimum alignment with the sequence SEQ ID NO: 14, or with a sequence as defined in b) said polypeptide having a DNA polymerase activity and/or DNA primase activity and/or nucleotidyl transferase activity, preferably a DNA polymerase activity at a temperature comprised between 70°C and 80°C.

6. A nucleic acid encoding a polypeptide according to one of claims 1 to 5.

7. A vector comprising the nucleic acid of claim 6.

8. The vector of claim 7, wherein said nucleic acid is operably linked to a promoter.

9. A host cell comprising the vector of claims 7 and 8.

10. A method of producing a DNA polymerase, said method comprising:
(a) culturing the host cell of claim 9 in conditions suitable for the expression of said nucleic acid; and
(b) isolating said DNA polymerase from said host cell.

11. A method of producing a recombinant DNA polymerase which is soluble in an aqueous solvent, said method comprising:
a) culturing the host cell of claim 9 in conditions suitable for the expression of said nucleic acid;
b) harvesting the cells by centrifugation and resuspending the cells pellet in an aqueous buffer;
c) lysing the cells, preferably by sonication, and, optionally, heating the lysate; and
d) centrifugating and recovering the aqueous soluble fraction containing the recombinant DNA polymerase.

12. The method of claim 11, wherein said host cell is a prokaryotic or an eukaryotic cell.

13. A method of synthesizing a double-stranded DNA molecule comprising:
(a) hybridizing a primer to a first DNA molecule; and
(b) incubating said DNA molecule of step (a) in the presence of one or more deoxyribonucleoside triphosphates or analogs thereof and the polypeptide of one of claims 1 to 5 or obtained by a method of claim 10 or 11, under conditions sufficient to synthesize a second DNA molecule complementary to all or a portion of said first DNA molecule.

14. A method of synthesizing a double-stranded DNA molecule from an unknown DNA template:
(a) incubating said DNA template in the presence of one or more deoxyribonucleoside triphosphates or analogs thereof and the polypeptide of one of claims 1 to 5 or obtained by a method of claim 10 or 11 having a DNA polymerase and primase activities, under conditions sufficient to synthesize a second DNA molecule complementary to all or a portion of said first DNA molecule.

15. A method for production of DNA molecules of greater than 10 kilobases in length comprising the methods of claims 12 or 13, wherein the first DNA molecule: which serve as a template is greater than 10 kilobases.

16. The method of claims 13 to 15, wherein said deoxyribonucleoside triphosphates are selected from the group consisting of dATP, dCTP, dGTP and dTTP.

17. A method for amplifying a double stranded DNA molecule, comprising:
(a) providing a first and second primer, wherein said first primer is complementary to a sequence at or near the 3'-termini of the first strand of said DNA molecule and said second primer is complementary to a sequence at or near the 3'-termini of the second strand of said DNA molecule;
(b) hybridizing said first primer to said first strand and said second primer to said second strand in the presence of the polypeptide of claims 1 to 3, under conditions such that a nucleic acid complementary to said first strand and a nucleic acid complementary to said second strand are synthesized;
(c) denaturing
- said first and its complementary strands; and
- said second and its complementary strands; and
(d) repeating steps (a) to (c) one or more times.

18. A method of preparing cDNA from mRNA, comprising:
(a) contacting mRNA with an oligo(dT) primer or other complementary primer to form a hybrid, and
(b) contacting said hybrid formed in step (a) with the DNA polymerase polypeptide of claims 1 to 5 or obtained by a method of claims 10 to 13, dATP, dCTP, dGTP and dTTP, whereby a cDNA-RNA hybrid is obtained.

19. A method of preparing dsDNA from mRNA, comprising:
(a) contacting mRNA with an oligo (dT) primer or other complementary primer to form a hybrid; and
(b) contacting said hybrid formed in step (a) with the polypeptide of claims 1 to 5 or obtained by a method of claims 10 to 13, dATP, dCTP, dGTP and dTTP, and an oligo nucleotide or primer which is complementary to the first strand cDNA;
whereby dsDNA is obtained.

20. A method for determining the nucleotide base sequence of a DNA molecule, comprising the steps of:
(a) contacting said DNA molecule with a primer molecule able to hybridize to said DNA molecule;
(b) incubating said hybrid formed in step (a) in a vessel containing four different deoxynucleoside triphosphates, a DNA polymerase polypeptide of claims 1 to 5 or obtained by a method of claims 10 to 13, and one or more DNA synthesis terminating agents which terminate DNA synthesis at a specific nucleotide base, wherein each said agent terminates DNA synthesis at a different nucleotide base; and
(c) separating the DNA products of the incubating reaction according to size, whereby at least a part of the nucleotide base sequence of said DNA can be determined.

21. The method of claim 20, wherein said terminating agent is a dideoxynucleoside triphosphate.

22. A method for amplification of a DNA molecule comprising the steps of:
(a) incubating said DNA molecule in the presence of a DNA polymerase polypeptide of claims 1 to 5 or obtained by a method of claims 10 to 13 and a mixture of different deoxynucleoside triphosphates.

23. A method according to one of claims 13 to 22, wherein an enzyme having a helicase activity is added in order to improve the processivity of the DNA polymerase polypeptide of claims 1 to 5 or obtained by a method of claims 10 to 13.

24. A kit for sequencing a DNA molecule, comprising:
(a) a first container means comprising a DNA polymerase polypeptide of claims 1 to 5 or obtained by a method of claims 10 to 13;
(b) a second container means comprising one or more dideoxyribonucleoside triphosphates; and
(c) a third container means comprising one or more deoxyribonucleoside triphosphates.

25. A kit for amplifying a DNA molecule, comprising:
(a) a first container means comprising DNA polymerase polypeptide of claims 1 to 5 or obtained by a method of claims 10 to 13; and
(b) a second container means comprising one or more deoxyribonucleoside triphosphates.

26. Use of a DNA polymerase polypeptide of claims 1 to 5 or obtained by a method of claims 10 to 13, for rolling circle amplification, multiple displacement amplification or protein-primed amplification.
